# EUROPÄISCHE PATENTSCHRIFT

(11) **EP 3 153 240 B1**
(45) Veröffentlichungstag und Bekanntmachung des Hinweises auf die Patenterteilung: **01.08.2018**
(21) Anmeldenummer: 16187677.6
(22) Anmeldetag: 07.09.2016
(51) Int. Cl.: B05C 17/005, B05C 17/015, B05C 17/01

(54) **PASTEN-APPLIKATIONSVORRICHTUNG ZUM MISCHEN EINER PASTE**
PASTE APPLICATION DEVICE FOR MIXING A PASTE
SYSTÈME D'APPLICATION DE PÂTE DESTINÉ AU MÉLANGE D'UNE PÂTE

(30) Priorität: 09.10.2015 DE 102015117270
(43) Veröffentlichungstag der Anmeldung: 12.04.2017
(73) Patentinhaber: Heraeus Medical GmbH, 61273 Wehrheim (DE)
(72) Erfinder: Vogt, Sebastian, 99092 Erfurt (DE); Kluge, Thomas, 56179 Vallendar (DE)
(74) Vertreter: Schultheiss & Sterzel Patentanwälte PartG mbB

(56) Entgegenhaltungen:
- EP-A1- 3 050 634
- DE-B3-102014 113 816
- DE-U1- 8 716 355
- SU-A1- 1 271 581
- US-A- 5 893 486

## Beschreibung

Die Erfindung betrifft eine Pasten-Applikationsvorrichtung zum Lagern zweier Ausgangskomponenten, zum Mischen der Ausgangskomponenten zu einer Paste und zum Applizieren der Paste.

Die Erfindung betrifft ferner ein Verfahren zum Mischen und Austreiben einer Paste.

Gegenstand der Erfindung ist somit eine manuell zu bedienende Pasten-Applikationsvorrichtung, die zum Austragen von pastenförmigen Massen, insbesondere von pastenförmigem Polymethylmethacrylat-Zementteig (PMMA-Zementteig) bestimmt ist. Die Pasten-Applikationsvorrichtung und das Verfahren sind zudem zum Lagern, Vermischen und zum Austragen von pastenförmigen Zweikomponentensystemen vorgesehen. Die Vorrichtung ist nur zum einmaligen Gebrauch bestimmt.

Konventionelle Polymethylmethacrylat-Knochenzemente (PMMA-Knochenzemente) werden meist aus einer pulverförmigen Komponente und einer flüssigen Monomerkomponente hergestellt (K.-D. Kühn: Knochenzemente für die Endoprothetik: ein aktueller Vergleich der physikalischen und chemischen Eigenschaften handelsüblicher PMMA-Zemente. Springer-Verlag Berlin Heidelberg New York, 2001). Diese PMMA-Knochenzemente werden nach Vermischung des Zementpulvers mit der flüssigen Monomerkomponente im noch nicht ausgehärteten, pastenförmigen Zustand als Zementteig appliziert. Bei Verwendung von Mischsystemen befindet sich der Zementteig im Fall von Pulver-Flüssigkeitszementen in einer Kartusche. Aus dieser Kartusche wird der Zementteig durch Bewegung eines Förderkolbens heraus gedrückt. Eine solche Bewegung des Förderkolbens kann mit Hilfe einer mechanischen Applikationsvorrichtung bewirkt werden.

Bei pastenförmigen Zweikomponenten-Pasten beziehungsweise Zweikomponenten-Knochenzementen, wie beispielsweise aus der DE 10 2007 050 762 B3, der DE 10 2008 030 312 A1 oder der DE 10 2007 052 116 B4 bekannt, werden beide pastenförmigen Komponenten in zwei separaten Kartuschen mit zwei separaten Förderkolben aufbewahrt. Bei der Applikation werden beide Pasten durch Bewegung der Förderkolben aus den Kartuscheninnenräumen in einen statischen Mischer gepresst und nach erfolgter Vermischung durch ein Austragsrohr ausgetragen.

Die Applikation von pastenförmigen Kleb- und Dichtstoffen erfolgt in der prinzipiell gleichen Weise mit Pasten-Applikationsvorrichtungen.

Zum Auspressen von zähen viskosen Massen werden gegenwärtig Pasten-Applikationsvorrichtungen verwendet, die manuell oder pneumatisch oder auch elektrisch angetrieben werden. Übliche einfache mechanische Pasten-Applikationsvorrichtungen nutzen insbesondere Klemmstangen zum Auspressen, die durch einen manuell zu betätigenden Kipphebel angetrieben werden. Bei hochviskosen Pasten sind diese Vorrichtungen nur mit sehr großem Kraftaufwand zu bedienen. Dieser Kraftaufwand ist medizinischen Anwendern im OP nicht zu zumuten.

Elektrisch angetriebene Auspressvorrichtungen können sowohl mit Akkumulatoren beziehungsweise mit Batterien als auch mit Hilfe einer stationären Stromversorgung angetrieben werden. Diese Vorrichtungen können mit ihren zum Teil sehr großen Kräften besonders zähe pastenförmige Massen auspressen. Nachteilig ist jedoch bei der Verwendung von Elektromotoren, dass diese Buntmetalle enthalten und kostenintensiv sind. Zudem müssen Akkumulatoren vorgehalten werden oder es ist ein im OP-Bereich hinderlicher Kabelanschluss vorzusehen, mit dem die Pasten-Applikationsvorrichtung an ein Stromnetz angeschlossen sein muss.

Pneumatische Pasten-Applikationssysteme, wie beispielsweise die aus der US 2 446 501 A, der DE 20 2005 010 206 U1, der US 2004 074 927 A1 oder der US 6 935 541 B1 bekannten Systeme, erfordern einen Druckluftanschluss. Dazu sind Druckluftschläuche notwendig, welche die Bewegung des Anwenders und die Anwendung des Pasten-Applikationssystems behindern können. Alternativ dazu ist auch die Verwendung von Druckgaspatronen zur Bereitstellung von Druckgas möglich. Exemplarisch dafür seien die Dokumente US 2 818 999 A, EP 3 050 634 A1 und EP 1 118 313 A1 genannt. Dazu wurden Vorrichtungen vorgeschlagen (in den oben genannten Druckschriften US 2004 074 927 A1 und US 6 935 541 B1), bei denen der Druckgaszufluss durch ein Ventil und zusätzlich durch ein zweites Ventil der Strom der viskosen Masse gesteuert werden. Bei diesen Vorrichtungen sind die Gaspatronen in den Vorrichtungen integriert. Nachteilig ist bei diesen Vorrichtungen, dass zwei Ventile verwendet werden.

Ein interessanter Vorschlag ist in der US 4 925 061 A1 beschrieben. Bei diesem System ist keine Gaspatrone in der Vorrichtung angeordnet. Die Vorrichtung muss über ein Ventil vor der Anwendung mit Druckgas aufgefüllt werden. In dieser Vorrichtung ist eine Kartusche enthalten, in der die viskose Masse angeordnet ist. Hinter der viskosen Masse ist ein Kolben angeordnet, der mit einem Faltenbalg mit einem Ende der Kartusche verbunden ist. In dem Hohlraum, der von dem Kolben, dem Faltenbalg und dem Kartuschenende gebildeten wird, wird vor der Anwendung flüssiges Gas durch ein Ventil eingefüllt. Dieser Vorschlag ist kritisch zu sehen, weil bei der Expansion von Flüssiggas eine Abkühlung eintritt, die zur Versprödung des elastischen Faltenbalgs führen kann. Bei einer Versprödung des Faltenbalgs sind Undichtigkeiten nicht auszuschließen. Dadurch ist es dann möglich, dass Druckgas durch den Faltenbalg austritt und neben dem Kolben in die viskose Masse eintritt. Bei Polymethylmethacrylat-Knochenzementpasten ist eine Vermischung der Pasten mit Druckgas nicht akzeptabel. Gasblasen würden zu einer Schwächung des ausgehärteten Polymethylmethacrylat-Knochenzementes führen. Weiterhin ist das Befüllen mit flüssigem Druckgas kompliziert und medizinischen Anwendern im OP nicht zu zumuten.

Mit dem Patent DE 10 2010 019 223 B4 wird eine Zementiervorrichtung vorgeschlagen, bei der durch Bewegung eines Drehventils eine Druckgaspatrone angestochen wird. Das Druckgas drückt direkt auf die Kolben der Vorrichtung. Der Austrag der Pasten wird durch ein Ventil reguliert. Nachteilig ist an der Vorrichtung, dass hochfeste Kunststoffe für die Kartuschen verwendet werden müssen, weil kein Druckbehälter vorgesehen ist.

Grundsätzlich kann es bei allen druckgasgetriebenen Pasten-Applikationsvorrichtungen mit Förderkolben passieren, dass sich Druckgas ungewollt an den Förderkolben vorbei in die Pasten presst. Je höher der Gasdruck ist, desto stärker kann sich dieses Problem ausprägen.

Weiterhin sind aus dem medizinischen Bereich Vorrichtungen zum Austrag von Medikamenten bekannt, die auch Druckgaspatronen als Energiequelle nutzen, bei denen jedoch keine Regulierung des Volumenstroms des Medikaments über Ventile vorgesehen ist (US 2008 086 079 A1, US 2008 208 114 A1). Solche Systeme sind für Knochenzemente nicht gut verwendbar, da sie keine gezielte Portionierung des Knochenzementteigs während der OP erlauben.

Die Aufgabe der Erfindung besteht darin, die Nachteile des Stands der Technik zu überwinden. Insbesondere soll eine Pasten-Applikationsvorrichtung und ein Verfahren zum Mischen und Austreiben einer Paste gefunden werden, mit dem auch sehr zähe Ausgangskomponenten mit einem einfachen Aufbau gelagert, gemischt und ausgetragen werden können. Die Pasten-Applikationsvorrichtung soll möglichst einfach bedienbar sein, wobei vorzugsweise nur eine Hand zum Halten und Bedienen der Pasten-Applikationsvorrichtung notwendig sein soll. Dabei soll das Pasten-Applikationssystem ausreichend kostengünstig sein, um eine einmalige Anwendung (Wegwerfartikel) zu ermöglichen. Wegwerfartikel sind aufgrund der hohen hygienischen Anforderungen im OP-Bereich besonders vorteilhaft. Die Pasten-Applikationsvorrichtung soll auch möglichst kompakt aufgebaut sein, damit sie einfach zu handhaben ist.

Die Aufgabe der Erfindung besteht auch darin, die Nachteile der bisher bekannten Pasten-Applikationsvorrichtungen zum Lagern, Vermischen und Applizieren von pastenförmigen Polymethylmethacrylat-Knochenzementen zu überwinden, bei denen die Energie zum Vermischen und Applizieren von einer Druckgaspatrone bereitgestellt wird, die in der Vorrichtung integriert ist. Die Vorrichtung soll mit nur einer Hand gehalten werden können, wobei der Austrag des gemischten Zementteigs mit der gleichen Hand gesteuert werden soll, mit der die Vorrichtung gehalten wird. Die normale Handkraft soll zum Betätigen ausreichen. Dabei soll ein zuverlässiges starten (beziehungsweise anhalten) und stoppen des Austrags des Knochenzements möglich sein.

Eine Aufgabe der Erfindung ist dabei ferner darin zu sehen, eine Pasten-Applikationsvorrichtung bereit zu stellen, die ohne Verwendung von externen stationären Energiequellen, wie Druckluft oder elektrischen Strom, angetrieben werden kann und die in der Lage ist, zähe pastöse Massen auszupressen und zu mischen. Die Pasten-Applikationsvorrichtung soll auch kein Kupfer und auch keine Kupferlegierungen enthalten. Sie soll ferner manuell durch den Anwender ortsunabhängig angewendet werden können und einen maximal vereinfachten Aufbau besitzen. Es soll eine Vorrichtung entwickelt werden, die zum Lagern, Vermischen und Austragen von hochviskosen pastenförmigen Polymethylmethacrylat-Knochenzement geeignet ist, wobei als Energiequelle Druckgas verwendet werden soll, das in einer Druckgaspatrone gelagert wird, die in der Vorrichtung integriert ist. Ferner soll es möglich sein, eine kostengünstige Pasten-Applikationsvorrichtung bereit zu stellen, die nur zum einmaligen Gebrauch bestimmt ist. Weiterhin soll die Erfindung auch die Aufgabe lösen, ein Verfahren zum Austragen von pastenförmigen Massen mit der zu entwickelnden Pasten-Applikationsvorrichtung zu entwickeln.

In der Pasten-Applikationsvorrichtung soll weiterhin vor der Zementapplikation eine separate Lagerung von zwei Zementpasten möglich sein. Die Oberfläche der Pasten-Applikationsvorrichtung soll weiterhin mit Ethylenoxid zu sterilisieren sein. Wichtig ist, dass während der Applikation durch den erforderlichen hohen Druck keine unerwünschten Zementaustritte möglich sind, die zur Kontamination des medizinischen Anwenders und des Operations-Saals (OP-Saals) führen könnten. Die Pasten-Applikationsvorrichtung sollte möglichst mit geringem Kraftaufwand manuell zu betätigen sein. Für die Anwendung ist es weiterhin wichtig, dass beim Stoppen der Bedienung der Pasten-Applikationsvorrichtung, der Zementteig beziehungsweise die Paste nicht in größeren Mengen nachfließt.

Es soll ferner eine Vorrichtung entwickelt werden, die zum Lagern, Vermischen und Austragen von hochviskosen pastenförmigen Polymethylmethacrylat-Knochenzement geeignet ist, wobei als Energiequelle Druckgas verwendet werden soll, das in einer Druckgaspatrone gelagert wird, die in der Pasten-Applikationsvorrichtung integriert ist. Eine weitere Aufgabe besteht darin, dass die Pasten-Applikationsvorrichtung hohe Gasdrücke von mehr als 30 bar über einen Anwendungszeitraum von wenigen Minuten sicher mechanisch toleriert, ohne dass sich die Pasten-Applikationsvorrichtung deformiert oder zerstört wird. Die zu entwickelnde Pasten-Applikationsvorrichtung soll zudem eine kostengünstige Fertigung erlauben. Weiterhin soll die Pasten-Applikationsvorrichtung möglichst keine den Druckgasfluss regulierende Ventile enthalten und auch keine separaten Vorrichtungen, die zur Verdampfung von flüssigem Druckgas bestimmt sind. Die zu entwickelnde Pasten-Applikationsvorrichtung muss so robust aufgebaut sein, dass das Druckgas mit verflüssigtem Druckgas vermischt sein kann. Bei Verwendung von flüssigem Kohlendioxid als Druckgas muss die Pasten-Applikationsvorrichtung auch Gemische aus gasförmigen, flüssigen und auch festem Kohlendioxid ohne mechanische Beschädigung oder Deformierung tolerieren.

Die Aufgaben der Erfindung werden gelöst durch eine Pasten-Applikationsvorrichtung zum Lagern zweier Ausgangskomponenten, zum Mischen der Ausgangskomponenten zu einer Paste und zum Applizieren der Paste aufweisend
eine Zweikomponentenkartusche aufweisend zwei Innenräume, zwei in den Innenräumen verschiebbare Förderkolben, die die Innenräume auf einer ersten Seite der Zweikomponentenkartusche begrenzen, und zumindest zwei Austragsöffnungen, durch die die Innenräume an einer der ersten Seite gegenüberliegenden zweiten Seite der Zweikomponentenkartusche geöffnet sind,
einen Hohlzylinder aus einem Kunststoff mit einer offenen Stirnseite und einer bereichsweise geschlossenen Stirnseite, in dem sich ein axial beweglicher Kolben mit zumindest zwei daran befestigten Stößeln befindet, wobei die zumindest zwei Stößel in Richtung der offenen Stirnseite ausgerichtet sind, wobei der Hohlzylinder mit der offenen Stirnseite axial an der ersten Seite der Zweikomponentenkartusche anliegend angeordnet ist und wobei der Kolben gasdicht mit den Innenwänden des Hohlzylinders abschließt,
wobei die Zweikomponentenkartusche und der Hohlzylinder in einem Druckbehälter angeordnet sind,
wobei der Hohlzylinder an der bereichsweise geschlossenen Stirnseite einen Anschluss für eine Druckgaspatrone aufweist, der sich durch eine Öffnung im Druckbehälter erstreckt,
wobei in Fließrichtung hinter den Austragsöffnungen ein manuell drehbares Klappenventil angeordnet ist oder ein Austragsrohr mit einem manuell drehbaren Klappenventil zu befestigen ist, wobei mit dem angeordneten Klappenventil der Volumenfluss der Ausgangskomponenten durch die Austragsöffnungen zu regulieren ist,
wobei das Klappenventil über ein Gestänge der Pasten-Applikationsvorrichtung bedienbar ist, wobei das Gestänge in einer Hauptrichtung beweglich zum Hohlzylinder gelagert ist und das in wenigstens einer Richtung senkrecht zur Hauptrichtung mit einem Spiel von zumindest 0,3 mm gelagert ist.

Dass der Volumenfluss der Ausgangskomponenten durch die Austragsöffnungen zu regulieren ist, bedeutet insbesondere, dass der Volumenfluss durch Schließen des Klappenventils zu stoppen ist.

Die Innenräume der Zweikomponentenkartusche sind vorzugsweise zylindrisch aufgebaut und die Förderkolben sind axial in den Innenräumen verschiebbar angeordnet.

Unter einer Zylindergeometrie wird erfindungsgemäß die allgemeine Zylinderform mit beliebiger Grundform verstanden, also nicht nur Zylinder mit kreisförmiger Grundfläche.

Vorzugsweise ist das Gestänge in einer Führung in der Hauptrichtung beweglich zum Hohlzylinder geführt.

Erfindungsgemäß kann bevorzugt vorgesehen sein, dass das manuell bedienbare Klappenventil über einen Abzug an einem Griff der Pasten-Applikationsvorrichtung bedienbar ist.

Es wird erfindungsgemäß bevorzugt, wenn die Pasten-Applikationsvorrichtung mit einer Hand zu halten ist und das Klappenventil mit der gleichen Hand zu bedienen ist. Hierdurch ist eine vereinfachte Anwendung der Pasten-Applikationsvorrichtung möglich.

Die Innenräume sind vorzugsweise mit zwei zu mischenden Ausgangskomponenten gefüllt, von denen zumindest eine eine Paste ist, wobei besonders bevorzugt die gemischten Ausgangskomponenten einen PMMA-Knochenzement bilden. Das Gemisch soll mit der Pasten-Applikationsvorrichtung aufzutragen sein.

Vorzugsweise schließt der Kolben nicht nur gasdicht mit den Innenwänden des Hohlzylinders ab, sondern sogar druckdicht bis zumindest 10 Atmosphären beziehungsweise bis zumindest 1000 kPa, besonders bevorzugt druckdicht bis zumindest 3000 kPa.

Bevorzugt ist das Gestänge in wenigstens einer Richtung senkrecht zur Hauptrichtung mit einem Spiel von zumindest 1 mm gelagert, besonders bevorzugt mit einem Spiel von zumindest 2 mm gelagert.

Bevorzugt ist ferner das Klappenventil außerhalb des Druckbehälters angeordnet. Die Achse des Klappenventils besteht erfindungsgemäß bevorzugt aus Stahl, um die Kräfte, die auf die Klappe wirken, ohne Verformung oder Zerstörung aufnehmen zu können.

Gemäß einer besonders bevorzugten Weiterbildung der vorliegenden Patentanmeldung kann vorgesehen sein, dass die Pasten-Applikationsvorrichtung einen manuell bedienbaren Abzug umfasst, mit dem das Gestänge in der Hauptrichtung bewegbar ist und dadurch das Klappenventil bedienbar ist.

Hiermit wird erreicht, dass die Pasten-Applikationsvorrichtung leicht bedienbar ist. Insbesondere dann, wenn ein Griff nach Art eines Pistolengriffs an der Unterseite der Pasten-Applikationsvorrichtung vorgesehen ist und sich der Abzug in Reichweite der Finger einer Hand des Anwenders befindet, kann die Pasten-Applikationsvorrichtung bequem mit einer Hand bedient werden, so dass die andere Hand während der OP für andere Tätigkeiten frei beziehungsweise verfügbar bleibt.

Dabei kann vorgesehen sein, dass der Abzug zwei voneinander beabstandete Hebel aufweist, zwischen die zumindest ein Finger einer Hand greifen kann, so dass der Abzug mit dem zumindest einen Finger der Hand in beide Richtungen bewegbar ist, wobei der erste Hebel durch Ziehen des Abzugs bedient wird und dabei das Klappenventil öffnet und der zweite Hebel durch eine umgekehrtes Drücken des Abzugs bedient wird und dabei das Klappenventil schließt.

Der Abstand zwischen den beiden Hebeln des Abzugs beträgt zumindest 1,5 cm, damit der zumindest eine Finger der Hand eines Anwenders zwischen die Hebel greifen kann, vorzugsweise zumindest 3 cm.

Hiermit wird erreicht, dass der Abzug nicht nur zum Öffnen des Klappenventils verwendet werden kann, sondern durch umgekehrten Druck auf den Abzug mittels des zweiten Hebels auch wieder manuell mit der gleichen Hand geschlossen werden kann.

Ferner kann vorgesehen sein, dass der Abzug mit einem Hub von maximal 100 mm das Klappenventil vom geschlossenen in den maximal geöffneten Zustand überführt, vorzugsweise mit einem Hub zwischen 2 mm und 40 mm, besonders bevorzugt mit einem Hub zwischen 5 mm und 15 mm.

Der maximal geöffnete Zustand des Klappenventils ist der Zustand, in dem das Klappenventil mit Hilfe der Pasten-Applikationsvorrichtung maximal geöffnet wurde. Es kann sein, dass sich das Klappenventil außerhalb der Pasten-Applikationsvorrichtung noch weiter öffnen lassen würde, dies aber nicht mit Hilfe der Pasten-Applikationsvorrichtung möglich ist.

Ein derartiger Hub lässt sich noch mit einer Bewegung der Finger einer Hand erzeugen, so dass die Pasten-Applikationsvorrichtung einfach zu bedienen ist.

Gemäß einer weiteren bevorzugten Ausgestaltung der vorliegenden Erfindung kann vorgesehen sein, dass zumindest ein Rückstellelement auf das Gestänge und/oder das Klappenventil einwirkt, so dass das Rückstellelement das Klappenventil in die geschlossene Position dreht, wobei insbesondere das Rückstellelement eine Druckfeder ist, die sich an einem Teil eines Gehäuses abstützt, das mit dem Hohlzylinder verbunden ist, wobei vorzugsweise das zumindest eine Rückstellelement durch Ziehen des Abzugs zu spannen ist.

Hiermit wird erreicht, dass das Klappenventil der Pasten-Applikationsvorrichtung mit dem Rückstellelement in Richtung der geschlossenen Position gedreht wird, wenn der Anwender nicht mehr den Abzug beziehungsweise den ersten Hebel in die Stellung zum Öffnen des Klappenventils zieht. Der zweite Hebel des Abzugs ist auch hier von großem Vorteil, da durch leichtes Antippen des zweiten Hebels mit der Fingerrückseite ein Anfangswiderstand, wie beispielsweise eine Haftreibung, überwunden werden kann und auch ein zusätzlicher Druck zum Schließen des Klappenventils, das heißt zur Überwindung des letzten Widerstands über den zweiten Hebel und das Gestänge auf das Klappenventil, ausgeübt werden kann.

Dabei kann vorgesehen sein, dass das Rückstellelement eine Druckfeder ist, die sich an einem Teil eines Gehäuses abstützt, das mit dem Hohlzylinder verbunden ist.

Diese Ausführung ist besonders einfach und kostengünstig zu realisieren.

Des Weiteren kann vorgesehen sein, dass der Druckbehälter an dem Hohlzylinder anliegt oder einen Abstand von höchstens 0,4 mm zum Hohlzylinder, bevorzugt von höchstens 0,1 mm aufweist, besonders bevorzugt von höchstens 100 µm aufweist, ganz besonders bevorzugt von höchstens 50 µm aufweist.

Auch bei einem geringem Abstand von höchstens 0,4 mm zwischen dem Druckbehälter und dem Hohlzylinder kann der Druckbehälter die Druckkräfte aufnehmen, da bei der Entspannung des Gases aus der Druckgaspatrone, die beim Einleiten des Druckgases aus der Druckgaspatrone in der Hohlzylinder erfolgt, eine Expansion (teilweise adiabatische Expansion) stattfindet, bei der das sich entspannende Gas abkühlt, diese die umgebenden Behältnisse abkühlen und sich infolge dessen der Hohlzylinder aus Kunststoff weniger stark zusammenzieht als der Druckbehälter, wenn dieser aus Metall oder Metalllegierung gefertigt ist und somit sich der metallische Druckbehälter an den Hohlzylinder anlegt. Ein geringer Abstand von maximal 0,1 mm oder weniger wird jedoch bevorzugt, um eine geringere Verformung und einen festeren Sitz des Druckbehälters zu ermöglichen. Bei Abständen von höchstens 100 µm kann der Druckbehälter der Pasten-Applikationsvorrichtung den Druck ohne größere Verformung des Hohlzylinders und der Zweikomponentenkartusche gut aufnehmen. Die Außenwandung der Zweikomponentenkartusche besteht vorzugsweise aus Kunststoff. Die Förderkolben können ebenfalls aus Kunststoff gefertigt werden, wobei bevorzugt zusätzlich umlaufende Dichtungen zum Abdichten der Förderkolben gegen die Innenräume an den Förderkolben angeordnet sind. An den Förderkolben können zudem Abstreiflippen angeordnet sein, mit denen der Inhalt der Innenräume vollständig oder zumindest zu wenigstens 99% aus den Innenräumen ausgetrieben werden kann.

Um einen kompakteren Aufbau der Pasten-Applikationsvorrichtung zu erreichen, kann vorgesehen sein, dass an dem Anschluss für die Druckgaspatrone eine Druckgasleitung angeschlossen ist, wobei die Druckgasleitung den Anschluss mit der Druckgaspatrone verbindet oder mit einer Öffnungsvorrichtung für die Druckgaspatrone verbindet, wobei vorzugsweise die Druckgasleitung ein durch Gewebe verstärkter Kunststoffschlauch ist.

Hierdurch ist es möglich, die Druckgaspatrone an der Unterseite oder im Griff der Pasten-Applikationsvorrichtung unterzubringen, so dass diese nicht zu sperrig und unhandlich wird und dadurch die Anwendbarkeit beeinträchtigt wird.

Bevorzugte Pasten-Applikationsvorrichtungen können sich auch dadurch auszeichnen, dass der Druckbehälter aus Metall, einer metallischen Legierung, einem hochfesten Kunststoff, einem faserverstärkten Kunststoff oder einer Kombination daraus gefertigt ist, wobei bevorzugt der Druckbehälter aus Metall oder einer metallischen Legierung besteht.

Dabei kann bevorzugt vorgesehen sein, dass der Druckbehälter aus Aluminium, Zink, einer Aluminiumlegierung oder Stahl besteht.

Metalle oder Metalllegierungen sind als Material für den Druckbehälter bevorzugt, da viele metallische Werkstoffe trotz hoher Druckfestigkeit beziehungsweise trotz hoher Zugfestigkeit leicht zu bearbeiten sind. Als hochfeste Kunststoffe für den Druckbehälter kommen Duroplaste, Polyamide, Polyamide-co-imide, Polysulfone, Polyketone und Polyetherketone in Frage, die allerdings schwerer als Metalle oder Metalllegierungen zu bearbeiten sind und daher als Material für den Druckbehälter weniger bevorzugt sind. Als faserverstärkte Kunststoffe sind Glasfaser-verstärkte Kunststoffe besonders bevorzugt.

Es kann ferner vorgesehen sein, dass an der Drehachse des Klappenventils ein Joch oder ein Bogen kraftschlüssig oder formschlüssig befestigt ist, das oder der mit dem Gestänge über eine Achse verbunden ist.

Hiermit wird eine gute Kraftübertragung auf die Drehachse des Klappenventils erreicht, so dass die Pasten-Applikationsvorrichtung zuverlässig bedienbar ist.

Dabei kann vorgesehen sein, dass die Achse, mit dem das Joch oder der Bogen mit dem Gestänge verbunden ist, parallel zur Drehachse des Klappenventils angeordnet ist, wobei vorzugsweise diese Achse am Scheitelpunkt des Bogens oder des Jochs angeordnet ist.

Damit wird eine symmetrische Kraftübertragung erreicht und der Hebel, den das Joch oder der Bogen bildet, optimal genutzt. Zudem werden ungleichmäßige mechanische Belastungen der Pasten-Applikationsvorrichtung im Bereich des Klappenventils auf diese Art vermieden.

Gemäß einer vorteilhaften Weiterbildung der vorliegenden Erfindung kann auch vorgesehen sein, dass das Gestänge zweiteilig aufgebaut ist, wobei die beiden Teile des Gestänges über ein Befestigungsmittel, insbesondere über einen Bolzen, Schieber oder ein Rastelement, miteinander verbunden oder verbindbar sind, vorzugsweise formschlüssig miteinander verbunden oder verbindbar sind, wobei bevorzugt ein erster Teil des Gestänges mit dem Austragsrohr verbunden ist und ein zweiter Teil des Gestänges mit dem Hohlzylinder verbunden ist.

Hiermit wird erreicht, dass das Klappenventil in einem separaten Teil, insbesondere in einem Austragsrohr, angeordnet sein kann, das erst kurz vor dem Einsatz der Pasten-Applikationsvorrichtung an der restlichen Pasten-Applikationsvorrichtung befestigt werden kann. Damit ist es möglich, einen Verschluss vorzusehen, der vor der Anwendung durch das Austragsrohr ersetzt werden kann, wobei der Verschluss eine Abdichtung der Austragsöffnungen bewirkt, so dass die Pasten-Applikationsvorrichtung auch zum längerfristigen Lagern der Ausgangskomponenten geeignet ist.

Es wird auch vorgeschlagen, dass an der Pasten-Applikationsvorrichtung ein Griff angeordnet ist, mit dem die Pasten-Applikationsvorrichtung mit einer Hand haltbar ist, wobei das Gestänge, insbesondere über den Abzug, mit der gleichen Hand bedienbar ist und damit das Klappenventil mit der gleichen Hand zu Öffnen und zu schließen ist.

Hiermit wird eine einfache Bedienbarkeit der Pasten-Applikationsvorrichtung erreicht.

Dabei kann vorgesehen sein, dass der Abzug im geschlossenen Zustand des Klappenventils maximal 30 mm von dem Griff entfernt ist und der Abzug im maximal geöffneten Zustand des Klappenventils an dem Griff anliegt oder einen Abstand von nicht mehr als 10 mm hat.

Voraussetzung hierfür ist, dass das Klappenventil auch über den Abzug bedient wird.

Der Vorteil hieran ist, dass die Pasten-Applikationsvorrichtung leicht mit einer Hand gehalten und bedient werden kann.

Ferner kann dabei vorgesehen sein, dass die Druckgaspatrone oder die Druckgaspatrone und eine Öffnungsvorrichtung zum Öffnen der Druckgaspatrone in dem Griff angeordnet ist.

Hiermit können die äußeren Abmessungen der Pasten-Applikationsvorrichtung gering gehalten werden. Zudem kann die Druckgaspatrone in der Pasten-Applikationsvorrichtung selbst geöffnet werden.

Bei erfindungsgemäßen Pasten-Applikationsvorrichtungen kann auch vorgesehen sein, dass das Klappenventil in einer Leitung angeordnet ist, die durch ein Austragsrohr gebildet ist, wobei bevorzugt im Austragsrohr zusätzlich ein statischer Mischer vorgesehen ist, mit dem die Ausgangskomponenten beim Durchströmen des Austragsrohrs durch den statischen Mischer durchmischbar sind.

Hierdurch kann ein besonders einfacher Aufbau erreicht werden, der kostengünstig realisiert werden kann, der gleichzeitig für Fehlfunktionen unanfällig ist und der zum Regulieren der Ströme der Ausgangskomponenten mit großem Druck auch stabil genug ist.

Des Weiteren kann vorgesehen sein, dass der Druckbehälter zweiteilig aufgebaut ist, wobei die beiden Teile durch Vernietung, durch Verschraubung und/oder durch eine Überwurfmutter kraftschlüssig verbunden sind, wobei vorzugsweise ein erster Teil der beiden Teile des Druckbehälters die Zweikomponentenkartusche beinhaltet und der zweite Teil der beiden Teile des Druckbehälters den Hohlzylinder.

Hierdurch werden die Herstellung und die Montage der Pasten-Applikationsvorrichtung vereinfacht. Insbesondere die Befüllung der Zweikomponentenkartusche mit den Ausgangskomponenten wird hierdurch deutlich vereinfacht. Bevorzugt sind die beiden Teile des Druckbehälters formschlüssig und/oder stoffschlüssig miteinander verbunden, besonders bevorzugt über eine Falz miteinander verbunden.

Bevorzugte Ausgestaltungen der Erfindung können sich dadurch auszeichnen, dass die Zweikomponentenkartusche zumindest bereichsweise eine Koaxialkartusche ist, wobei in der Koaxialkartusche einer der Innenräume zylindrisch und innenliegend ist und der andere Innenraum zylindrisch ist und den innenliegenden Innenraum koaxial umschließt.

Die Förderkolben sind der Innenform der zylindrischen Innenräume angepasst. Die Zylinderform hat den Vorteil, dass sich die auftretenden großen Kräfte beim Auspressen der Ausgangskomponenten beziehungsweise beim Beaufschlagen der Zweikomponentenkartusche mit dem Druck aus der Druckgaspatrone gleichmäßig vom Druckbehälter aufgenommen werden können. Zudem können Bauteile mit zylindrischer Geometrie kostengünstig gefertigt werden.

Um eine Lagerung der Ausgangskomponenten des Knochenzements zu ermöglichen, kann vorgesehen sein, dass die Pasten-Applikationsvorrichtung einen Verschluss zum Verschließen der Austragsöffnungen aufweist, der mit einem Befestigungsmittel des Verschlusses, insbesondere mit einem Gewinde des Verschlusses, an einem Gegenbefestigungsmittel im Bereich der Austragsöffnungen, insbesondere an einem Gegengewinde im Bereich der Austragsöffnungen, zu befestigen ist, wobei vorzugsweise an dem Gegenbefestigungsmittel das Austragsrohr zu befestigen ist, das zu diesem Zweck ebenfalls ein dementsprechendes Befestigungsmittel aufweist.

Hierdurch können die Ausgangskomponenten länger in der Pasten-Applikationsvorrichtung gelagert werden, da mit dem Verschluss ein besserer und luftdichterer Abschluss der Innenräume mit den Ausgangskomponenten darin erreicht werden kann, als wenn nur das Ventilelement die Innenräume abdichten würde.

Dabei kann vorgesehen sein, dass der Verschluss ein Schlüssel zum Öffnen der Druckgaspatrone und/oder zum Verbinden der Druckgaspatrone mit einem Öffnungsmittel zum Öffnen der Druckgaspatrone ist, wobei vorzugsweise der Verschluss als Schlüssel bodenseitig auf die Druckgaspatrone zu stecken ist und damit die Druckgaspatrone zum Öffnen der Druckgaspatrone und/oder Verbinden der Druckgaspatrone mit dem Öffnungsmittel zum Öffnen der Druckgaspatrone bewegbar, insbesondere drehbar und/oder in Längsrichtung verschiebbar ist.

Hierdurch kann ein frühzeitiges versehentliches Öffnen der Druckgaspatrone vermieden werden, so dass die Bedienung der Pasten-Applikationsvorrichtung vereinfacht wird.

Es kann erfindungsgemäß auch vorgesehen sein, dass an dem Anschluss des Hohlzylinders oder an der Druckgasleitung ein Druckgaspatronenbefestigungsmittel vorgesehen ist, mit dem die Druckgaspatrone an dem Anschluss oder an der Druckgasleitung druckdicht zu befestigen ist, und/oder an dem Anschluss des Hohlzylinders oder der Druckgasleitung ein Stechdorn als Öffnungsmittel zum Öffnen der Druckgaspatrone vorgesehen ist oder das Öffnungsmittel einen solchen Stechdorn aufweist.

Durch diesen Aufbau wird eine sichere und dichte Verbindung erreicht beziehungsweise eine frühzeitige ungewollte Öffnung der Druckgaspatrone vermieden. Das Druckgaspatronenbefestigungsmittel ist vorzugsweise ein Innengewinde, in das ein Außengewinde der Druckgaspatrone eingeschraubt werden kann. An einer Verbindungsfläche kann zudem eine Dichtung (beispielsweise in Form eines O-Rings) vorgesehen sein, mit dem eine bessere Abdichtung der Verbindung der Druckgaspatrone zum Hohlzylinder erreicht wird.

Zur Verbesserung der Lagerungsmöglichkeit kann ferner vorgesehen sein, dass zwischen dem Hohlzylinder und der Zweikomponentenkartusche eine Schutzfolie angeordnet ist, die die Innenräume der Zweikomponentenkartusche auf der ersten Seite verschließen, wobei die Schutzfolie vorzugsweise auf die Zweikomponentenkartusche aufgeklebt, angeschweißt oder angehaftet ist.

Mit der Schutzfolie kann eine bessere Abdichtung der Innenräume erreicht werden, so dass eine längere beziehungsweise qualitätswahrende Lagerung von Ausgangskomponenten mit flüchtigen Bestandteilen, wie beispielsweise Methylmethacrylat, in den Innenräumen möglich wird. Die Schutzfolie wird im Betrieb beim Vortreiben der Stößel durch den Kolben im Hohlzylinder durchstoßen, so dass die Stößel die Förderkolben in der Zweikomponentenkartusche vortreiben können und damit die Ausgangskomponenten aus der Zweikomponentenkartusche durch die Austragsöffnungen aus den Innenräumen der Zweikomponentenkartusche austreiben können.

Bevorzugt ist die Schutzfolie eine Aluminium-Verbundfolie. Aluminium beziehungsweise Aluminium-beschichtete Folien sind gegenüber Methylmethacrylat-Dämpfen besonders dicht, so dass die Pasten-Applikationsvorrichtung dann besonders gut zum Lagern der Ausgangskomponenten eines PMMA-Knochenzements geeignet ist.

Es kann erfindungsgemäß bevorzugt vorgesehen sein, dass der Druckbehälter ein Zug-Modul nach EN ISO 527 größer 1500 MPa aufweist.

Es kann ferner erfindungsgemäß bevorzugt vorgesehen sein, dass der Druckbehälter eine Druckfestigkeit von mindestens 3 MPa aufweist.

Es kann auch vorgesehen sein, dass der Druckbehälter einen zylindrischen Innenraum zur Aufnahme des Hohlzylinders und der Zweikomponentenkartusche aufweist. Die zylindrische Geometrie ist sowohl zur Aufnahme der Kräfte besonders gut geeignet, als auch in der Fertigung leicht herzustellen.

Mit einer Weiterentwicklung wird vorgeschlagen, dass in der Außenwand des Hohlzylinders, und zwar in der Hälfte, die in Richtung der Zweikomponentenkartusche ausgerichtet ist, und in der Wandung des Druckbehälters zumindest je eine durchgehende Entlüftungsöffnung vorgesehen ist, so dass der Druck aus dem Hohlzylinder entweicht, wenn der Kolben zwischen der Entlüftungsöffnungsöffnung und der Zweikomponentenkartusche angeordnet ist.

Hierdurch wird die Pasten-Applikationsvorrichtung nach dem Auspressen der Ausgangskomponenten druckfrei und kann anschließend gefahrlos recycelt oder entsorgt werden. Durch die Entlüftungsöffnungen ist der Raum zwischen der Zweikomponentenkartusche und den axial beweglichen Stößeln im Hohlzylinder mit der umgebenden Atmosphäre gasdurchlässig verbunden. Dabei kann vorgesehen sein, dass bei Erreichen der Endposition des Kolbens im Hohlzylinder das Druckgas in die Umgebung entweicht.

Bevorzugt ist die zumindest eine Entlüftungsöffnung des Hohlzylinders in der Zylindermantelwand des Hohlzylinders angeordnet. Ebenso bevorzugt ist die zumindest eine Entlüftungsöffnung des Druckbehälters in der Zylindermantelwand des Druckbehälters angeordnet. Es kann besonders bevorzugt auch vorgesehen sein, dass die Entlüftungsöffnungen im Hohlzylinder und im Druckbehälter einander überdeckend angeordnet sind. An der zumindest einen Entlüftungsöffnung des Druckbehälters kann ein manuell bedienbares Ventilelement zum Ablassen der Druckluft angeordnet sein.

Gemäß einer Weiterbildung kann auch vorgesehen sein, dass die Zweikomponentenkartusche, der Hohlzylinder und der Druckbehälter in einem Gehäuse angeordnet sind, wobei bevorzugt auch die Druckgaspatrone in dem Gehäuse angeordnet ist und/oder das Gehäuse an einer Unterseite der Pasten-Applikationsvorrichtung als Griff ausgebildet ist.

Hierdurch wird die Pasten-Applikationsvorrichtung nach außen abgeschlossen. Das Gehäuse besteht bevorzugt aus Kunststoff.

Die der vorliegenden Erfindung zugrundeliegenden Aufgaben werden auch gelöst durch ein Verfahren zum Mischen und Austreiben einer Paste gekennzeichnet durch die Schritte:
ein Druckgas wird aus einer Druckgaspatrone durch einen Anschluss eines Hohlzylinders in den Hohlzylinder geleitet, wobei ein Druckbehälter die auf den Wänden des Hohlzylinders lastende Kraft des Druckgases aufnimmt und ein Kolben mit Stößeln im Hohlzylinder durch das Druckgas in Richtung einer Zweikomponentenkartusche vorangetrieben wird;
die vorangetriebenen Stößel des Kolbens treiben Förderkolben in zumindest zwei Innenräume der Zweikomponentenkartusche voran, wobei die Ausgangskomponenten aus den Innenräumen der Zweikomponentenkartusche durch die zumindest zwei Austragsöffnungen der Innenräume ausgetrieben werden, wobei der Fluss der Ausgangskomponenten durch ein geschlossenes Klappenventil in Fließrichtung hinter den Austragsöffnungen gestoppt wird und durch eine manuelle Bedienung eines Bedienelementes ein Joch oder ein Bogen gedreht werden und dabei die mit dem Joch oder dem Bogen verbundene Drehachse des Klappenventils gedreht wird, so dass das Klappenventil geöffnet wird und die Ausgangskomponenten und/oder deren Mischung durch das geöffnete Klappenventil strömen und nach einem Mischen der Ausgangskomponenten die Mischung appliziert wird,
wobei durch die manuelle Bedienung des Bedienelements ein Gestänge bewegt wird und durch diese Bewegung ein Joch oder ein Bogen gedreht wird. Dabei wird die mit dem Joch oder dem Bogen verbundene Drehachse des Klappenventils gedreht.

Es kann dabei vorgesehen sein, dass die beim Einleiten des Druckgases in den Hohlzylinder und die beim Vortreiben des Kolbens und der Förderkolben in der Zweikomponenten-Kartusche auftretenden Kräfte durch den Druckbehälter aufgenommen werden, insbesondere durch einen im wesentlichen zylindrischen Druckbehälter aufgenommen werden, der die Zweikomponentenkartusche und den Hohlzylinder umschließt, wobei der Druckbehälter besonders bevorzugt an der Zweikomponentenkartusche und dem ersten Hohlzylinder anliegt.

Bevorzugt kann vorgesehen sein, dass zur Umsetzung des Verfahrens eine erfindungsgemäße Pasten-Applikationsvorrichtung verwendet wird.

Ferner kann vorgesehen sein, dass das Klappenventil durch die Einwirkung einer Kraft eines Rückstellelements, insbesondere einer elastischen Feder, und/oder durch eine manuelle Bedienung des Bedienelements in zur Bedienung zum Öffnen des Klappenventils umgekehrter Richtung geschlossen wird.

Erfindungsgemäß kann auch vorgesehen sein, dass vor dem Einleiten des Druckgases ein Verschluss von der Pasten-Applikationsvorrichtung entfernt wird, der die Austragsöffnungen der Innenräume der Zweikomponentenkartusche verschließt, und ein Austragsrohr vor dem Austragsöffnungen der Innenräume befestigt wird, in dem das Klappenventil angeordnet ist. Dabei kann bevorzugt vorgesehen sein, dass die Druckgaspatrone nach dem Befestigen des Austragsrohrs geöffnet wird und besonders bevorzugt mit dem Öffnen der Druckgaspatrone das Druckgas in den Hohlzylinder geleitet wird.

Der Erfindung liegt die überraschende Erkenntnis zugrunde, dass es mit Hilfe eines Druckbehälters und eines Klappenventils, das in Fließrichtung hinter den Kartuschen (den Innenräumen der Zweikomponentenkartusche) angeordnet ist und das mit viskosen Ausgangskomponenten gefüllt ist, gelingt, einen direkten Antrieb der Förderkolben mit dem Gasdruck aus einer Druckgaspatrone zu erzeugen, ohne dass der Druck des Gases zuvor reduziert werden müsste. Der Druckbehälter ist in der Lage, die großen auftretenden Kräfte aufzunehmen, ohne dass sich die innenliegenden Kunststoff-Formkörper (die Zweikomponentenkartusche und der Hohlzylinder) verformen oder zu stark verformen. Ferner kann das Klappenventil trotz der hohen auftretenden Kräfte über ein Gestänge bedient werden, indem die Drehachse des Klappenventils mit Hilfe eines Hebels bedient wird, auf den das Gestänge einwirkt. Hierzu weist die Lagerung des Gestänges senkrecht zur Hauptbewegungsrichtung ein Spiel von zumindest 0,3 mm auf, mit dem die Drehbewegung des Hebels aufgenommen wird. Die innenliegenden Kunststoff-Formkörper sollten aus Kunststoff gefertigt sein, damit die Antriebselemente (der Kolben und die Förderkolben) gut in den Kunststoff-Formkörpern gleiten können und gleichzeitig abdichten. Das Klappenventil muss dazu ausreichend stabil konstruiert werden. Die Positionierung in einem relativ engen Kanal hinter den Innenräumen der Zweikomponentenkartusche hilft dabei, den Aufbau des Klappenventils ohne großen Konstruktionsaufwand stabil genug zu gestalten. Theoretisch kann das Klappenventil auch in Fließrichtung hinter einem statischen Mischer angeordnet werden, der die auf das Klappenventil wirkende Kraft dann weiter reduziert. Der Aufbau kann so relativ einfach und funktionell gestaltet werden, ohne dass aufwendige Bauteile zur Leitung und Beeinflussung des Druckgasflusses notwendig wären. Durch die Reduzierung der Anzahl der Bauteile wird auch die Anfälligkeit der Pasten-Applikationsvorrichtung für Fehlfunktionen oder Störungen erheblich reduziert.

Bevorzugt ist die Drehachse des Klappenventils mit einem Bogen oder einem Joch kraft- oder formschlüssig verbunden. An dem Scheitelpunkt des Bogens oder des Jochs ist besonders bevorzugt eine Stange als Teil des Gestänges angelenkt, wobei die Stange parallel zur Längsachse der Vorrichtung an der Unterseite des Gehäuses angeordnet ist. Diese Stange ist drehbar um die Längsachse des Bogens oder des Jochs beweglich. Am Ende der Stange ist ein erster Hebel als Teil eines Abzugs senkrecht zur Stange angeordnet. Beim Zug des ersten Hebels mit dem Zeigefinger, Mittelfinger und Ringfinger in Richtung des Handgriffs zieht die Stange den Bogen oder das Joch ebenfalls in Richtung des Handgriffs. Dabei wird durch Drehung der Drehachse das Klappenventil geöffnet. Zum Schließen des Klappenventils wird mit der Rückseite des Zeigefingers, des Mittelfingers und des Ringfingers ein zweiter Hebel des Abzugs entgegengesetzt zum Handgriff bewegt. Beachtet werden muss dabei, dass sowohl zum Öffnen als auch zum Schließen des Klappenventils nur sehr kurze Wege von wenigen Millimetern zur Auslenkung des ersten Hebels und des zweiten Hebels möglich sind, damit eine komfortable Bedienung möglich ist.

Eigene Versuche mit Druckgas-getriebenen Pasten-Applikationsvorrichtungen zeigten, dass es entscheidend ist, dass die mit Druckgas beaufschlagten Bauteile gasdicht sind und sich nicht durch den Druck derart stark deformieren, dass die Funktion der Pasten-Applikationsvorrichtung eingeschränkt würde. Dabei wurden Förderkolben einer Koaxialkartusche durch einen Antriebskolben bewegt, auf den ein Gasdruck von 30 bis 50 bar einwirkte. Dadurch wurde eine Auspresskraft im Bereich von 12 bis 18 kN ausgeübt. Der Volumenstrom wurde durch ein vorgelagertes Klappenventil gesteuert, das durch einen Hebel von 11 cm manuell bewegt werden konnte. Das Klappenventil ist durch eine Drehachse drehbar gelagert. Die Drehachse wird mit einer Kraft von mehreren Kilonewton in das Lager gedrückt. Durch diese hohe Anpressung der Drehachse ist die Haft- und Gleitreibung der Drehachse im Lager sehr hoch. Es zeigte sich dabei, dass infolge der sehr hohen Auspresskraft ein relativ hohes Drehmoment zur Steuerung des Klappenventils notwendig ist. Insbesondere ist eine Rückstellung des Klappenventils nach erfolgter Öffnung in den geschlossenen Ausgangszustand nur durch sehr starke Rückstellfedern möglich. Das bedeutet, dass bei Verwendung von starken Rückstellfedern zum Schließen des Klappenventils der Anwender zuvor beim Öffnen des Klappenventils einmal die Haft- und Gleitreibung und zusätzlich die Kraft der Rückstellfeder überwinden muss.

In weiteren Versuchen wurde gefunden, dass es sehr schwierig ist, Zementkartuschen während des Austrags der Zementpasten, während des Vorschubs der Förderkolben durch Stößel, so zu lagern, dass sich die Zementkartuschen bei Innendrücken größer 20 bar nicht von den Stößeln und dem Stößelantrieb durch Verbiegung oder Verwindung entfernen. Eigene Versuche zeigten weiterhin, dass aus Aluminiumlegierungen gefertigte Zementkartuschen für Polymethylmethacrylat-Zementpasten ungeeignet sind, weil die Legierungen und die darin enthaltenden Legierungsbestandteile wie Kupfer und Mangan eine unerwünschte radikalische Polymerisationen währen der Lagerung der Zementpasten in der Zementkartusche beziehungsweise den Innenräumen bewirken. Nur in Zementkartuschen aus geeigneten Kunststoffen, wie Poly-acrylnitril-co-methylmethacrylat und Polybutylenterephthalat, sind Polymethylmethacrylat enthaltende Zementpasten ausreichend dauerhaft lagerstabil.

Im Folgenden wird ein erstes sehr allgemeines Ausführungsbeispiel der Erfindung besch rieben:
Eine beispielhafte Pasten-Applikationsvorrichtung ist zusammengesetzt aus
   a) einem hohlzylinderförmiger ersten Druckbehälter mit einer Mindestdruckfestigkeit von 30 bar,
   b) mindestens einer Kunststoffkartusche mit einem axial beweglichen Austragskolben, die im ersten Druckbehälter angeordnet ist,
   c) einem Austragsrohr, das einen statischen Mischer und ein Klappenventil mit einer Drehachse enthält und das formschlüssig mit der Kunststoffkartusche verbunden ist, wobei das Klappenventil außerhalb des ersten Druckbehälters angeordnet ist,
   d) einen hohlzylinderförmiger zweiten Druckbehälter mit einer Mindestdruckfestigkeit von 30 bar, mit einer offenen Stirnseite und einer geschlossenen Stirnseite, wobei die geschlossene Stirnseite einen Durchbruch hat, wobei
   e) der Kunststoffhohlzylinder so im zweiten Druckbehälter angeordnet ist, dass ein Anschluss in den Hohlzylinder durch den Durchbruch des zweiten Druckbehälters ragt,
   f) der Spalt zwischen dem Kunststoffhohlzylinder und dem zweiten Druckbehälter kleiner 0,1 mm ist,
   g) ein axial beweglicher Kolben (Antriebskolben) im Kunststoffhohlzylinder angeordnet ist, wobei der Kolben mindestens mit einem Stößel verbunden ist,
   h) eine Öffnungsvorrichtung für eine Gaspatrone außerhalb des Druckbehälters angeordnet ist, die über eine gasdurchlässige Druckgasleitung mit der Öffnung des Hohlzylinders verbunden ist,
   i) die beiden Druckbehälter und formschlüssig oder stoffschlüssig miteinander verbunden sind, und
   j) einem geschlossenen Gehäuse, in dem der erste Druckbehälter, der zweite Druckbehälter, die Druckgasleitung, die Öffnungsvorrichtung der Druckgaspatrone und eine Druckgaspatrone angeordnet sind.

Es kann auch vorgesehen sein, dass die Pasten-Applikationsvorrichtung einen Kunststoffhohlzylinder mit einer offenen und einer geschlossenen Stirnseite aufweist, wobei an der Außenseite des Kunststoffhohlzylinders ein Hohlzylinder angeordnet ist, der mit dem Innenraum des Kunststoffhohlzylinders gasdurchlässig verbunden ist, und der an seiner Außenseite ein Außengewinde hat.

Derartige Pasten-Applikationsvorrichtungen können dadurch charakterisiert sein, dass
a) an der Unterseite der Vorrichtung das Gehäuse als Handgriff ausgebildet ist,
b) die beiden Enden der Drehachse des Klappenventils mit einem Bogen formschlüssig oder kraftschlüssig verbunden sind,
c) das am Scheitelpunkt des Bogens eine Stange (als Gestänge) angeordnet ist, die drehbar zur Längsachse des Bogens gelagert ist und die parallel zur Längsachse der Vorrichtung ausgerichtet ist,
d) einem ersten Hebel (als Teil des Abzugs) der am Ende des der Stange senkrecht zur Stangenachse angeordnet ist, wobei der erste Hebel im geschlossenen Zustand des Klappenventils 5 bis 15 mm von der Vorderseite des Handgriffs entfernt ist,
e) einem zweiten Hebel (als Teil des Abzugs), der mindestens 3,0 cm vom ersten Hebel entfernt senkrecht zur Hebelachse angeordnet ist,
f) eine Führung für den Stange an der Unterseite des Gehäuses vor dem Handgriff angeordnet ist und
g) im geöffneten Zustand des Klappenventils der erste Hebel einen Abstand von 0 bis 5mm zum Handgriff hat.

Erfindungsgemäß ist, dass die Stange zweitteilig ausgebildet ist, wobei die beiden Teile der Stange formschlüssig durch einen Schieber oder ein Rastelement verbunden sind. Das Austragsrohr mit dem Klappenventil, dem Bogen oder dem Joch und dem ersten Teil der Stange bildet eine Baugruppe. Der zweite Teil des Gestänges wird durch die Führung am Gehäuse gehalten. Nach Entfernung des Verschlusses der Kartusche wird die Baugruppe in die Kartusche verschraubt. Danach wird der erste Teil des Gestänges mit dem zweiten Teil des Gestänges durch einen Schieber kraftschlüssig verbunden, der an am zweiten Teil oder auch am ersten Teil des Gestänges angebracht ist. Alternativ ist es auch möglich, dass Rastelemente am ersten Teil und am zweiten Teil des Gestänges angeordnet sind, so dass beide Teile des Gestänges durch Verrastung miteinander verbunden werden können.

Vorteilhaft ist es, wenn an der Stange beziehungsweise dem Gestänge ein Federelement angeordnet ist, dass sich am Handgriff abstützt und das Gestänge in Richtung Bogen oder Joch drückt. Dadurch wird die manuelle Rückstellbewegung beim Schließen des Klappenventils erleichtert.

Der erste Druckbehälter und der zweite Druckbehälter sind aus Metall und/oder aus Kunststoff und/oder aus Glasfaser-verstärkten Kunststoffen gebildet, wobei die Druckbehälter bevorzugt aus Metall bestehen. Ganz besonders bevorzugt bestehen die Druckbehälter aus Aluminium. Die Behälter können vorteilhaft durch Fließpressen hergestellt werden.

Die Gaspatrone mit der Öffnungsvorrichtung ist vorteilhaft im Handgriff angeordnet. Dadurch kann die gesamte Vorrichtung sehr kurz gestaltet werden. Als Gaspatronen kommen besonders Gaspatronen in Betracht, die mit 16 g oder mit 24 g Kohlendioxid befüllt sind. Daneben ist auch die Verwendung von anderen nicht toxischen Gasen, wie Stickstoff, möglich.

Erfindungsgemäß kann auch vorgesehen sein, dass die Druckgasleitung durch einen Schlauch gebildet wird, der die Öffnungsvorrichtung der Druckgaspatrone mit der Öffnung des Hohlzylinders gasdurchlässig verbindet, wobei als Druckgasleitung ein durch Gewebe verstärkter Kunststoffschlauch bevorzugt wird. Alternativ sind auch Kunststoffschläuche verwendbar, die mit Metallgeflecht ummantelt sind.

Im Folgenden werden weitere Ausführungsbeispiele der Erfindung anhand von sieben schematisch dargestellten Figuren erläutert, ohne jedoch dabei die Erfindung zu beschränken. Dabei zeigt:
- Figur 1:: eine Querschnittansicht einer erfindungsgemäßen Pasten-Applikationsvorrichtung mit geschlossenem Klappenventil;
- Figur 2:: eine schematische Seitenansicht auf die Pasten-Applikationsvorrichtung nach Figur 1 mit geöffnetem Klappenventil;
- Figur 3:: eine perspektivische Ansicht der Pasten-Applikationsvorrichtung nach den Figuren 1 und 2 mit offenem Gehäuse und mit geöffnetem Klappenventil;
- Figur 4:: eine schematische perspektivische Querschnittansicht der Pasten-Applikationsvorrichtung nach den Figuren 1 bis 3 mit geöffnetem Klappenventil;
- Figur 5:: eine Querschnittansicht der erfindungsgemäßen Pasten-Applikationsvorrichtung nach Figur 1 mit geöffnetem Klappenventil;
- Figur 6:: eine perspektivische Ansicht der Pasten-Applikationsvorrichtung nach den Figuren 1 bis 5 mit offenem Gehäuse, separatem Austragsrohr und Verschluss; und
- Figur 7:: eine vergrößerte Teilansicht der Verbindung des Gestänges der Pasten-Applikationsvorrichtung nach Figuren 1 bis 6.

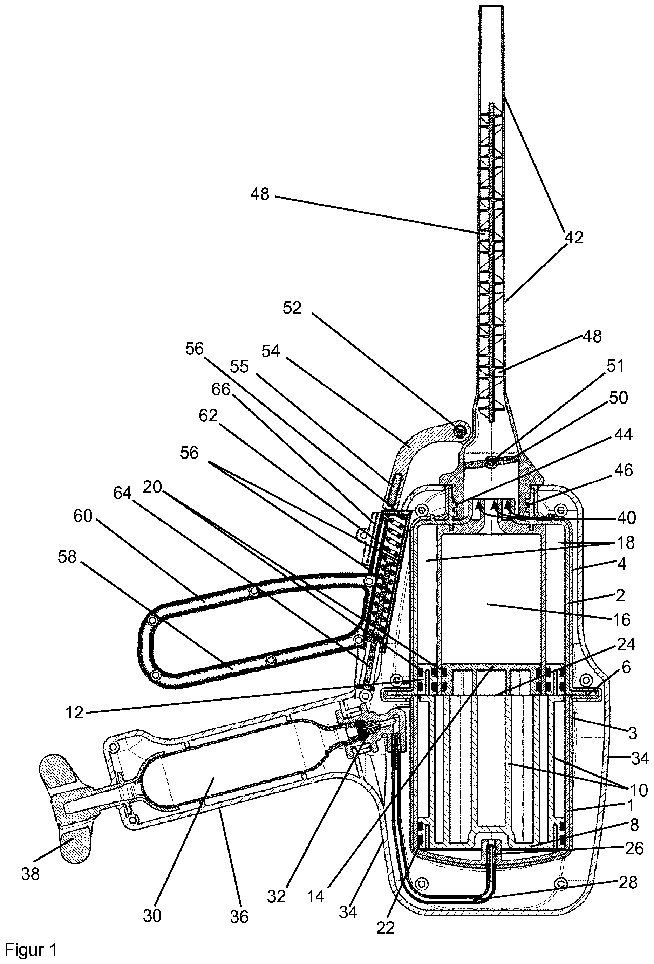

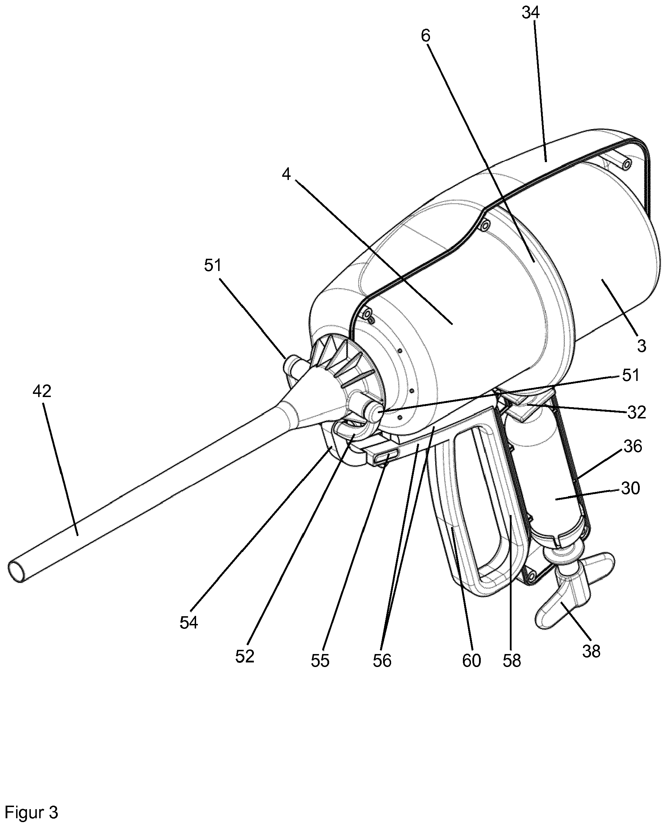

Die Figuren 1 bis 7 zeigen unterschiedliche Ansichten einer erfindungsgemäßen Druckgas-getriebenen Pasten-Applikationsvorrichtung beziehungsweise von Teilen davon. Die Pasten-Applikationsvorrichtung ist nach Art einer Pistole aufgebaut und kann in einer Hand gehalten und mit der gleichen Hand beziehungsweise den Fingern der gleichen Hand, beziehungsweise den Fingern der gleichen Hand bedient werden.

Im Inneren der Pasten-Applikationsvorrichtung befindet sich ein Hohlzylinder 1 aus Kunststoff und eine benachbart angeordnete Zweikomponentenkartusche 2. Der Hohlzylinder 1 ist von einem ersten Teil 3 eines Druckbehälters 3, 4 umschlossen und die Zweikomponentenkartusche 2 ist von einem zweiten Teil 4 des Druckbehälters 3, 4 umschlossen. Die beiden Teile des Druckbehälters 3, 4 bestehen aus einem metallischen Werkstoff, wie einer Aluminium-Legierung, Zink oder Stahl, und sind über eine Verbindung 6 formschlüssig durch eine nach außen gerichtete Falz umlaufend und stoffschlüssig miteinander verbunden. Die Außenwände des Hohlzylinders 1 und der Zweikomponentenkartusche 2 liegen an den Innenwänden des Druckbehälters 3, 4 flächig an oder weisen einen Anstand von maximal 100 µm auf, damit der Druckbehälter 3, 4 Kräfte, die auf die Wandungen des Hohlzylinders 1 und der Zweikomponentenkartusche 2 wirken, aufnehmen kann, ohne dass eine für die Funktion der Pasten-Applikationsvorrichtung störende Verformung der Wandungen erfolgt. Der Hohlzylinder 1, die Zweikomponentenkartusche 2 und der Druckbehälter 3, 4 haben eine zylindrische Form beziehungsweise eine im Wesentlichen zylindrische Form.

Im Inneren des Hohlzylinders 1 ist ein Kolben 8 als Arbeitskolben vorgesehen, mit dem die im Druckgas gespeicherte Energie in eine lineare Bewegung entlang der Zylinderachse beziehungsweise in Längsrichtung der Pasten-Applikationsvorrichtung umgewandelt werden kann. An dem Kolben 8 sind mehrere koaxial zueinander angeordnete Stößel 10 befestigt, die in Richtung der Zweikomponentenkartusche 2 ausgerichtet sind. Mit den Stößeln 10 können zwei Förderkolben 12, 14 angetrieben werden, die in koaxial zueinander angeordneten Innenräumen 16, 18 der Zweikomponentenkartusche 2 angeordnet sind. Die Förderkolben 12, 14 sind mit umlaufenden Dichtungen 20 gegen die Innenwände der Innenräume 16, 18 abgedichtet. Ebenso liegt der Kolben 8 umlaufend an der Innenwand des Hohlzylinders 1 an und ist dort mit umlaufenden Dichtungen 22 abgedichtet.

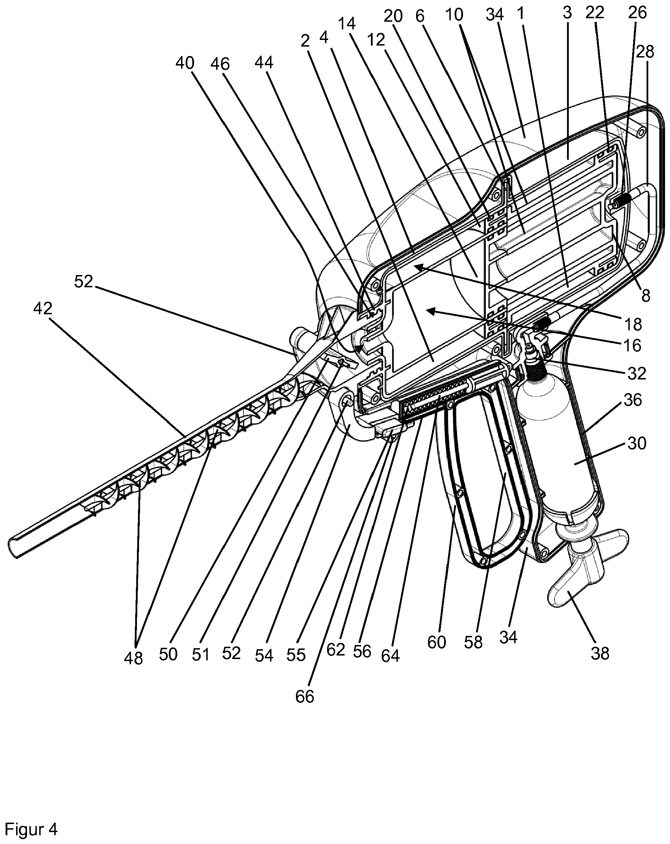

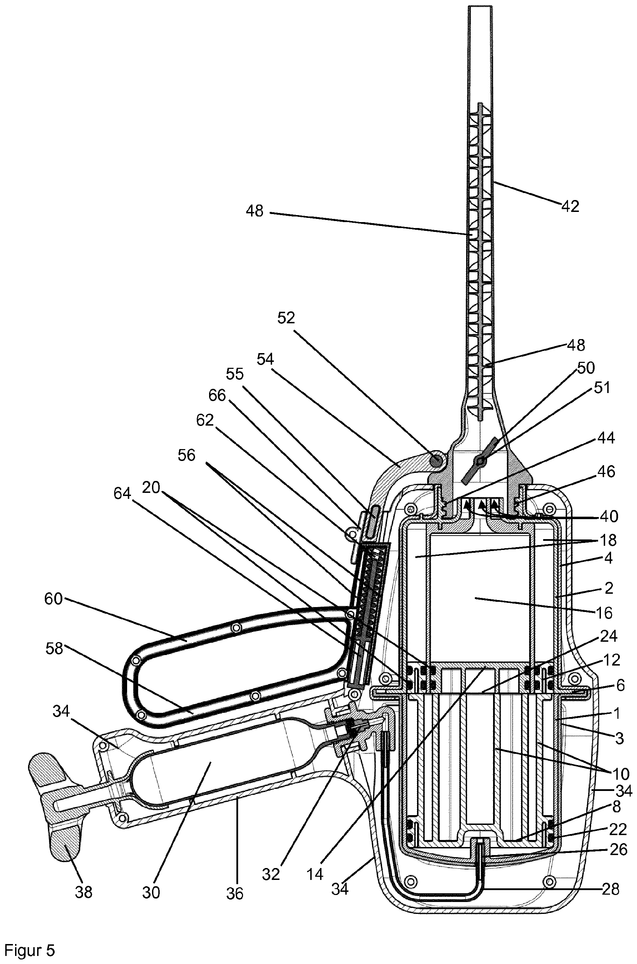

Zwischen dem Hohlzylinder 1 und den Stößeln 10 und der Zweikomponentenkartusche 2 ist eine Aluminium-Verbundfolie 24 auf die durch die Förderkolben 12, 14 geschlossenen Enden der Zweikomponentenkartusche 2 aufgeklebt. Die Aluminium-Verbundfolie 24 ist nur in Figur 1 und 5 eingezeichnet und in Figur 4 der Übersichtlichkeit wegen weggelassen. Durch die Aluminium-Verbundfolie 24 wird der Inhalt der Zweikomponentenkartusche 2 abgedichtet. Insbesondere wenn leicht flüchtige Bestandteile in den Ausgangskomponenten enthalten sind, die in den Innenräumen 16, 18 der Zweikomponentenkartusche 2 gelagert sind, wird ein entweichen dieser Bestandteile und damit eine Veränderung der Ausgangskomponenten bei der Lagerung vermieden. Beim Vortreiben der Förderkolben 12, 14 mit den Stößeln 10 des Kolbens 8 wird die Aluminium-Verbundfolie 24 einfach durchstoßen.

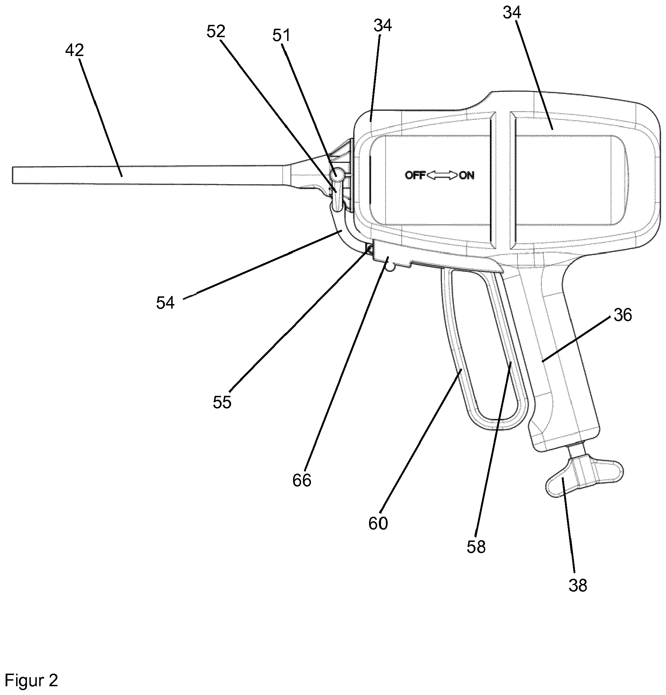

Das Druckgas mündet durch einen Anschluss 26 und eine Druckgasleitung 28 in den Hohlzylinder 1 zwischen der Rückseite des Kolbens 8 und der geschlossenen Seite des ersten Teils 3 des Druckbehälters 3, 4. Der Anschluss 26 ragt dazu durch die einzige Öffnung in der ansonsten geschlossenen Grundfläche des ersten Teils 3 des Druckbehälters 3, 4 in den Hohlzylinder 1 hinein. Der Hohlzylinder 1 und der erste Teil des Druckbehälters 3, 4 sind an der Rückseite (in den Figuren 1 und 5 unten, in den Figuren 2 bis 4 und 6 rechts) gewölbt gestaltet. Der Anschluss 26 sitzt als Hülse in einer Hülse des Hohlzylinders 1 und dient zum Anschließen der Druckgasleitung 28. Die Druckgasleitung 28 besteht aus einem faserverstärkten Kunststoff.

An die Druckgasleitung 28 ist eine Druckgaspatrone 30 angeschlossen, die geöffnet werden kann, indem die Druckgaspatrone 30 auf ein Öffnungsmittel 32 in Form eines Hohldorns 32 oder Stechdorns 32 gedrückt wird. Dadurch öffnet sich die Druckgaspatrone 30 und das Druckgas kann durch die Druckgasleitung 28 in den Anschluss 26 und in den Hohlzylinder 1 strömen und den Kolben 8 in Richtung der Zweikomponentenkartusche 2 vortreiben. Die Druckgaspatrone 30 ist bevorzugt eine Flüssiggas-Patrone, in der besonders bevorzugt Kohlendioxid verdampft, um das Druckgas bereitzustellen.

Der gesamte bisher genannte Aufbau beziehungsweise alle diese Teile (mit den Bezugszeichen 1 bis 32) sind innerhalb eines Gehäuses 34 aus Kunststoff angeordnet. Das Gehäuse 34 ist an der Unterseite (in Figur 1 und 5 links und in den Figuren 2, 3, 4 und 6 unten) als Griff 36 beziehungsweise Pistolengriff 36 ausgebildet, mit dem die Pasten-Applikationsvorrichtung gehalten werden kann. An der Druckgaspatrone 30 ist bodenseitig eine Bedienvorrichtung 38 befestigt, die in einer Flügelschraube endet, mit der die Druckgaspatrone 30 in das Anschlussstück ein passendes Gewinde an der Öffnungseinrichtung 32 eingeschraubt und durch den Hohldorn 32 oder Stechdorn 32 geöffnet werden kann. Die Pasten-Applikationsvorrichtung kann also manuell dadurch aktiviert werden beziehungsweise Startklar gemacht werden, dass die Bedienvorrichtung 38 bedient und damit die Druckgaspatrone 30 geöffnet wird.

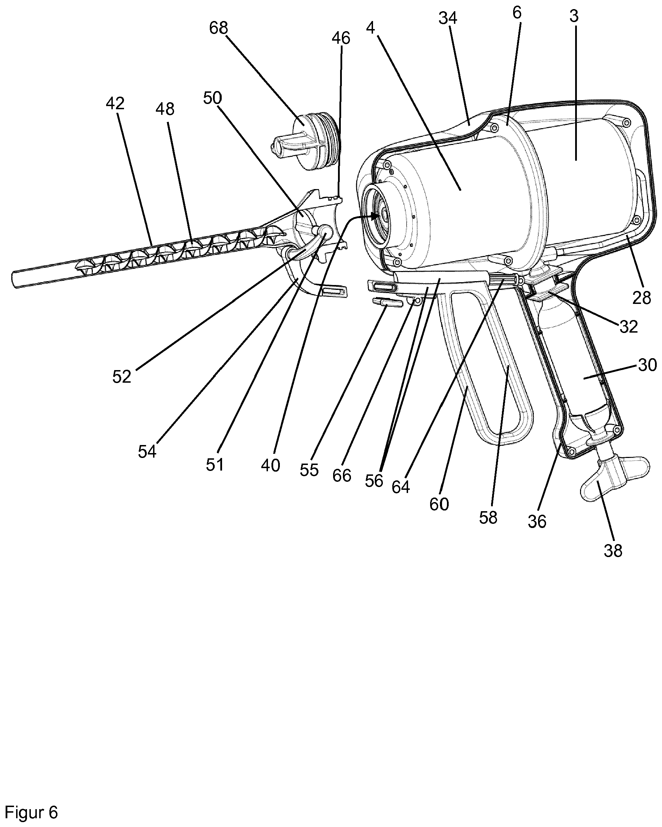

An der Vorderseite des zylindrischen Teils der Zweikomponentenkartusche 2 läuft diese und die koaxial angeordneten Innenräume 16, 18 darin konisch zusammen und münden in Austragsöffnungen 40, durch die die Ausgangskomponenten aus den Innenräumen 16, 18 aus der Zweikomponentenkartusche 2 ausgetrieben werden. In Fließrichtung hinter den Austragsöffnungen 40 mischen sich dann die Ausgangskomponenten. Die Austragsöffnungen 40 sind im Lagerungszustand der Pasten-Applikationsvorrichtung zunächst durch einen schraubbaren Verschluss 68 (nur in Figur 6 gezeigt) verschlossen. Im Anwendungszustand wird der Verschluss 68 entfernt und dafür ein Austragsrohr 42 an der Vorderseite befestigt. Dazu ist an der Vorderseite ein Innengewinde 44 vorgesehen, das in einem Stutzen an der Zweikomponentenkartusche 2 ausgebildet ist. In dieses Innengewinde 44 ist ein Außengewinde 46 des Austragsrohrs 42 geschraubt. Der Verschluss 68 weist ein analoges Außengewinde auf, das in das Innengewinde 44 der Zweikomponentenkartusche 2 geschraubt war beziehungsweise werden kann, um die Austragsöffnungen 40 zu verschließen.

Im Inneren des Austragsrohrs 42 ist ein statischer Mischer 48 vorgesehen, mit dem die Ausgangskomponenten durchmischt werden, wenn diese durch das Austragsrohr 42 strömen. Der Durchfluss durch das Austragsrohr 42 kann mit einem manuell bedienbaren Klappenventil 50 gesteuert werden, das im Kanal des Austragsrohrs 42 um eine Drehachse 51 drehbar gelagert ist. Wenn das Klappenventil 50 geschlossen ist, können die Ausgangskomponenten nicht weiter aus der Zweikomponentenkartusche 2 ausgetrieben werden und demzufolge die Förderkolben 12, 14 und der Kolben 8 nicht weiter vorangetrieben werden. Dadurch, dass nicht nur einfach der Gasstrom unterbrochen wird, wie bei anderen aus dem Stand der Technik bekannten Applikationsvorrichtungen, kann es nicht zu einer weiteren Expansion des bereits eingeleiteten Gases kommen und damit zu einem unerwünschten Nachlaufen der Pasten-Mischung. Die Anordnung des Klappenventils 50 in dem Kanal des Austragsrohrs 42 ist auch insofern geschickt, da aufgrund des geringen Querschnitts die Belastung der Lager des Klappventils 50 begrenzt ist.

In dem Hohlzylinder 1 und dem ersten Teil des Druckbehälters 3 können übereinander liegend mehrere Entlüftungsöffnungen (nicht zu sehen) vorgesehen sein. Die Entlüftungsöffnungen können beispielsweise in einer Höhe des Hohlzylinders 1 angeordnet sein, die zumindest und bevorzugt in etwa der Höhe des Kolbens 8 entspricht, so dass wenn der Kolben 8 bis zum Anschlag in Richtung der Zweikomponentenkartusche 2 vorgetrieben ist, der Bereich zwischen dem Kolben 8 und der Rückseite des Hohlzylinders 1 zur Umgebung durch die Entlüftungsöffnungen geöffnet wird, und der verbleibende Druck in die Umgebung entweichen kann. Hierdurch wird sichergestellt, dass die verbrauchte Pasten-Applikationsvorrichtung beim Recyceln oder bei der Entsorgung nicht mehr unter Druck steht und daher gefahrlos weiterverarbeitet beziehungsweise zerlegt werden kann.

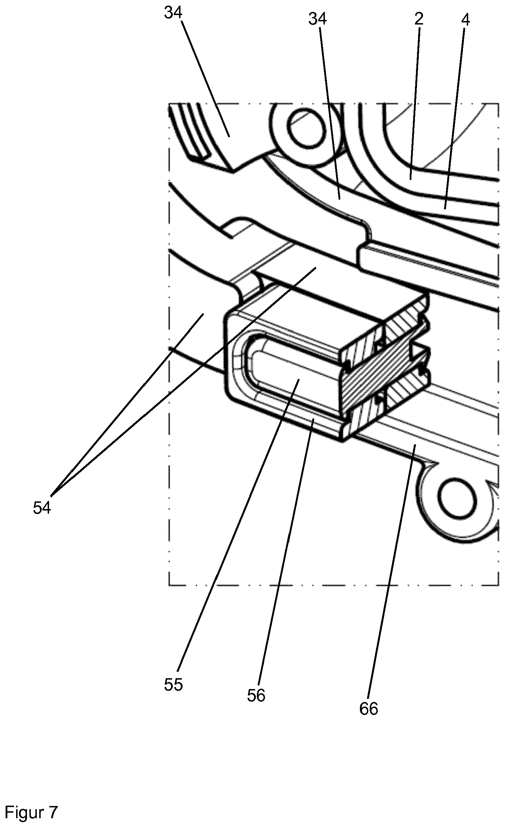

Das Klappventil 50 ist von außerhalb der Pasten-Applikationsvorrichtung bedienbar, indem ein Bogen 52 mit beiden Enden der Drehachse 51 des Klappenventils 50 verbunden ist, so dass der Bogen 52 das Klappenventil 50 umspannt. An dem Scheitelpunkt des Bogens 52 ist eine Stange 54 beziehungsweise ein Gestänge 54 drehbar mit dem Bogen 52 verbunden. Der Scheitelpunkt des Bogens 52 bildet die Achse, um die das Gestänge 54 drehbar gegen den Bogen 52 gelagert ist. Das Gestänge 54 erstreckt sich in Richtung des Griffs 36 und ist in dieser Hauptrichtung, nämlich der Längsrichtung des Gestänges 54, beweglich. Durch diese Bewegung kann das Klappenventil 50 über den Bogen 52 geöffnet und geschlossen werden. Das Gestänge 54 ist über ein Verbindungselement 55 in Form eines Schiebers 55 mit einem Rastelement (siehe Figur 7) mit einem hinteren Teil des Gestänges 56 verbunden. Das Rastelement dient dazu, dass sich der Schieber 55 nicht ungewollt aus den Ösen des Gestänges 54, 56 lösen kann und sich damit das Gestänge 54, 56 löst.

Diese Zweiteilung des Gestänges 54, 56 ermöglicht, dass das Klappenventil 50 als Teil des Austragsrohrs 42 erst kurz vor der Verwendung der Pasten-Applikationsvorrichtung an diese angebaut wird. Dadurch kann der Verschluss 68, der zur Lagerung der Ausgangskomponenten in den Innenräumen 16, 18 wesentlich besser geeignet ist, als das geschlossene Klappenventil 50, möglichst lange mit der Zweikomponentenkartusche 2 verbunden bleiben. Ohne den Verschluss 68 können die Ausgangskomponenten im Bereich vor den Austragsöffnungen 40 miteinander reagieren und aushärten, so dass die Pasten-Applikationsvorrichtung dann nicht mehr verwendbar wäre.

Das Gestänge 54, 56 ist mit einem Abzug 58, 60 bedienbar. Der Abzug 58, 60 weist zwei voneinander beabstandete Holme oder Hebel 58, 60 auf, die mit den Finger der Hand bedient werden können, mit der die Pasten-Applikationsvorrichtung an dem Griff 36 gehalten wird. Eine Stahlfeder 62 stützt sich als Rückstellelement 62 über eine Stange 64 an dem Gehäuse 34 im Bereich des Ansatzes des Griffs 36 ab und wirkt auf das Gestänge 34, 36 in der Art, dass das Gestänge 54, 56 vom Griff 36 weg gedrückt wird. Dadurch wird das Klappenventil 50 mit der Stahlfeder 62 in die geschlossene Stellung gedrückt. Durch Ziehen des Abzugs 58, 60 am ersten Teil 58 in Richtung des Griffs 36 wird das Klappenventil 50 geöffnet und die Stahlfeder 62 gespannt (siehe Figuren 2, 3, 4, 5).

Das Gestänge 56 ist in einer Führung 66 gelagert, die am Gehäuse 34 befestigt ist und damit starr mit dem Hohlzylinder 1 verbunden ist. Die Führung 66 gibt die Hauptrichtung der Bewegung des Gestänges 54, 56 vor. Aufgrund der Drehbewegung des Bogens 52 und der linearen Bewegung des Gestänges 54, 56 ist es notwendig, dass bei der Lagerung ein gewisses Spiel in einer Richtung senkrecht zur Hauptrichtung (in den Figuren 1 und 5 nach links und rechts, in den Figuren 2 bis 4 und 6 und 7 nach oben und unten) vorhanden ist. Ohne dieses Spiel würde das Gestänge 54, 56 entweder zu stark verbogen oder in der Führung 66 verkannten. Das Spiel sollte je nach Größe des Aufbaus der Pasten-Applikationsvorrichtung mindestens 0,3 mm oder bis zu mindestens 2 mm betragen.

Die gespannte Stahlfeder 62 drückt das Gestänge 54, 56 nach vorne und öffnet das Klappenventil 50, wenn keine Kraft mehr auf den ersten Teil 58 des Abzugs 58, 60 in Richtung des Griffs 36 wirkt. Zur Überwindung der Haftreibung und zum endgültigen Schließen des Klappenventils 50 kann eine zusätzliche Kraft aufgebracht werden, indem mit den Rückseiten der Finger der Hand, mit der auch der Griff 36 gehalten wird, ein Druck auf den zweiten Teil 60 des Abzugs 58, 60 in Richtung vom Griff 36 weg ausgeübt wird. Der Abzug 58, 60 ist dazu ausgelegt, dass mehrere Finger der Hand durch die Öffnung des Abzugs 58, 60 greifen, um den Abzug 58, 60 zu bedienen.

Im Bereich des Öffnungsmittels 32 ist ein Innengewinde vorgesehen, in das ein Außengewinde der Druckgaspatrone 30 geschraubt werden kann. Zwischen dem Anschluss und der Druckgaspatrone 30 beziehungsweise zwischen dem Hohldorn 32 und der Druckgaspatrone 30 ist eine Dichtung vorgesehen, um eine druckdichte Verbindung zu der Druckgasleitung 28 zu ermöglichen.

Die Drehachse 51 des Klappenventils 50 ist eine Stahlachse 51, um die das Klappenventil 50 in dem Austragsrohr 42 mit Hilfe des Bogens 52 drehbar ist. Die Stahlachse 51 sorgt für die nötige Stabilität des Klappenventils 50. Die Stahlachse 51 darf sich nämlich unter Einwirkung des Drucks aus der Druckgaspatrone 30 vermittelt durch die Ausgangskomponenten nicht derart plastisch Verformen, dass die Funktion des Klappenventils 50, beispielsweise die Beweglichkeit des Klappenventils 50, nicht mehr gewährleistet wäre. Durch Drehen des Klappenventils 50 um die Stahlachse 51 kann der Volumenfluss durch das Austragsrohr 42 gesteuert werden, während der Gasdruck vermittelt durch den Kolben 8 und die Förderkolben 12, 14 den Inhalt aus der Zweikomponentenkartusche 2 austreibt, das heißt, die Ausgangskomponenten aus der Zweikomponentenkartusche 2 austreibt.

Die in der voranstehenden Beschreibung, sowie den Ansprüchen, Figuren und Ausführungsbeispielen offenbarten Merkmale der Erfindung können sowohl einzeln, als auch in jeder beliebigen Kombination für die Verwirklichung der Erfindung in ihren verschiedenen Ausführungsformen wesentlich sein.

### Bezugszeichenliste

- 1: Hohlzylinder
- 2: Zweikomponentenkartusche
- 3: Druckbehälter, erster Teil
- 4: Druckbehälter, zweiter Teil
- 6: Verbindung
- 8: Kolben
- 10: Stößel
- 12: Förderkolben
- 14: Förderkolben
- 16: Innerer Innenraum
- 18: Äußerer Innenraum
- 20: Dichtung
- 22: Dichtung
- 24: Aluminium-Verbundfolie
- 26: Anschluss für Druckgas
- 28: Druckgasleitung
- 30: Druckgaspatrone
- 32: Öffnungsmittel mit Stechdorn / Hohldorn
- 34: Gehäuse
- 36: Griff
- 38: Bedienvorrichtung
- 40: Austragsöffnung
- 42: Austragsrohr
- 44: Innengewinde
- 46: Außengewinde
- 48: Statischer Mischer
- 50: Drehbares Klappenventil
- 51: Drehachse des Klappenventils
- 52: Bogen / Joch
- 54: Gestänge
- 55: Verbindungselement / Schieber
- 56: Gestänge / Stange
- 58: Abzug / erster Teil des Abzugs
- 60: Abzug / zweiter Teil des Abzugs
- 62: Feder / Rückstellelement
- 64: Stange
- 66: Führung
- 68: Verschluss

## Patentansprüche

1. Pasten-Applikationsvorrichtung zum Lagern zweier Ausgangskomponenten, zum Mischen der Ausgangskomponenten zu einer Paste und zum Applizieren der Paste aufweisend
eine Zweikomponentenkartusche (2) aufweisend zwei Innenräume (16, 18), zwei in den Innenräumen (16, 18) verschiebbare Förderkolben (12, 14), die die Innenräume (16, 18) auf einer ersten Seite der Zweikomponenten-kartusche (2) begrenzen, und zumindest zwei Austragsöffnungen (40), durch die die Innenräume (16, 18) an einer der ersten Seite gegenüberliegenden zweiten Seite der Zweikomponentenkartusche (2) geöffnet sind,
einen Hohlzylinder (1) aus einem Kunststoff mit einer offenen Stirnseite und einer bereichsweise geschlossenen Stirnseite, in dem sich ein axial beweglicher Kolben (8) mit zumindest zwei daran befestigten Stößeln (10) befindet, wobei die zumindest zwei Stößel (10) in Richtung der offenen Stirnseite ausgerichtet sind, wobei der Hohlzylinder (1) mit der offenen Stirnseite axial an der ersten Seite der Zweikomponentenkartusche (2) anliegend angeordnet ist und wobei der Kolben (8) gasdicht mit den Innenwänden des Hohlzylinders (1) abschließt, wobei die Zweikomponentenkartusche (2) und der Hohlzylinder (1) in einem Druckbehälter (3, 4) angeordnet sind,
wobei der Hohlzylinder (1) an der bereichsweise geschlossenen Stirnseite einen Anschluss (26) für eine Druckgaspatrone (30) aufweist, der sich durch eine Öffnung im Druckbehälter (3, 4) erstreckt,
wobei in Fließrichtung hinter den Austragsöffnungen (40) ein manuell drehbares Klappenventil (50) angeordnet ist oder ein Austragsrohr (42) mit einem manuell drehbaren Klappenventil (50) zu befestigen ist, wobei mit dem angeordneten Klappenventil (50) der Volumenfluss der Ausgangskomponenten durch die Austragsöffnungen (40) zu regulieren ist,
wobei das Klappenventil (50) über ein Gestänge (54, 56) der Pasten-Applikationsvorrichtung bedienbar ist, wobei das Gestänge (54, 56) in einer Hauptrichtung beweglich zum Hohlzylinder (1) gelagert ist und das in wenigstens einer Richtung senkrecht zur Hauptrichtung mit einem Spiel von zumindest 0,3 mm gelagert ist.

2. Pasten-Applikationsvorrichtung nach Anspruch 1, **dadurch gekennzeichnet, dass**
das Gestänge (54, 56) zweiteilig aufgebaut ist, wobei die beiden Teile des Gestänges (54, 56) über ein Befestigungsmittel (55), insbesondere über einen Bolzen, Schieber (55) oder ein Rastelement, miteinander verbunden oder verbindbar sind, vorzugsweise formschlüssig miteinander verbunden oder verbindbar sind.

3. Pasten-Applikationsvorrichtung nach Anspruch 2, **dadurch gekennzeichnet, dass**
ein erster Teil (54) des Gestänges (54, 56) mit dem Austragsrohr (42) verbunden ist und ein zweiter Teil (56) des Gestänges (54, 56) mit dem Hohlzylinder (1) verbunden ist.

4. Pasten-Applikationsvorrichtung nach einem der Ansprüche 1, 2 oder 3, **dadurch gekennzeichnet, dass**
zumindest ein Rückstellelement (62) auf das Gestänge (54, 56) und/oder das Klappenventil (50) einwirkt, so dass das Rückstellelement (62) das Klappenventil (50) in die geschlossene Position dreht, wobei vorzugsweise das zumindest eine Rückstellelement (62) durch Ziehen des Abzugs (58, 60) zu spannen ist.

5. Pasten-Applikationsvorrichtung nach einem der vorangehenden Ansprüche, **dadurch gekennzeichnet, dass**
die Pasten-Applikationsvorrichtung einen manuell bedienbaren Abzug (58, 60) umfasst, mit dem das Gestänge (54, 56) in der Hauptrichtung bewegbar ist und dadurch das Klappenventil (50) bedienbar ist.

6. Pasten-Applikationsvorrichtung nach Anspruch 5, **dadurch gekennzeichnet, dass**
der Abzug (58, 60) zwei voneinander beabstandete Hebel (58, 60) aufweist, zwischen die zumindest ein Finger einer Hand greifen kann, so dass der Abzug (58, 60) mit dem zumindest einen Finger der Hand in beide Richtungen bewegbar ist, wobei der erste Hebel (58) durch Ziehen des Abzugs (58, 60) bedient wird und dabei das Klappenventil (50) öffnet und der zweite Hebel (60) durch eine umgekehrtes Drücken des Abzugs (58, 60) bedient wird und dabei das Klappenventil (50) schließt.

7. Pasten-Applikationsvorrichtung nach Anspruch 5 oder 6, **dadurch gekennzeichnet, dass**
der Abzug (58, 60) mit einem Hub von maximal 100 mm das Klappenventil (50) vom geschlossenen in den maximal geöffneten Zustand überführt, vorzugsweise mit einem Hub zwischen 2 mm und 40 mm, besonders bevorzugt mit einem Hub zwischen 5 mm und 15 mm.

8. Pasten-Applikationsvorrichtung nach Anspruch 6, **dadurch gekennzeichnet, dass**
das Rückstellelement (62) eine Druckfeder (62) ist, die sich an einem Teil eines Gehäuses (34) abstützt, das mit dem Hohlzylinder (1) verbunden ist.

9. Pasten-Applikationsvorrichtung nach einem der vorangehenden Ansprüche, **dadurch gekennzeichnet, dass**
an der Drehachse (51) des Klappenventils (50) ein Joch oder ein Bogen (34) kraftschlüssig oder formschlüssig befestigt ist, das oder der mit dem Gestänge (54, 56) über eine Achse verbunden ist.

10. Pasten-Applikationsvorrichtung nach Anspruch 9, **dadurch gekennzeichnet, dass**
die Achse, mit dem das Joch oder der Bogen (34) mit dem Gestänge (54, 56) verbunden ist, parallel zur Drehachse (51) des Klappenventils (50) angeordnet ist, wobei vorzugsweise diese Achse am Scheitelpunkt des Bogens (34) oder des Jochs angeordnet ist.

11. Pasten-Applikationsvorrichtung nach einem der vorangehenden Ansprüche, **dadurch gekennzeichnet, dass**
an der Pasten-Applikationsvorrichtung ein Griff (36) angeordnet ist, mit dem die Pasten-Applikationsvorrichtung mit einer Hand haltbar ist, wobei das Gestänge (54, 56), insbesondere über den Abzug (58, 60), mit der gleichen Hand bedienbar ist und damit das Klappenventil (50) mit der gleichen Hand zu Öffnen und zu schließen ist.

12. Pasten-Applikationsvorrichtung nach Anspruch 13, **dadurch gekennzeichnet, dass**
der Abzug (58, 60) im geschlossenen Zustand des Klappenventils (50) maximal 30 mm von dem Griff (36) entfernt ist und der Abzug (58, 60) im maximal geöffneten Zustand des Klappenventils (50) an dem Griff (36) anliegt oder einen Abstand von nicht mehr als 10 mm hat.

13. Pasten-Applikationsvorrichtung nach einem der vorangehenden Ansprüche, **dadurch gekennzeichnet, dass**
das Klappenventil (50) in einer Leitung angeordnet ist, die durch ein Austragsrohr (42) gebildet ist, wobei bevorzugt im Austragsrohr (42) zusätzlich ein statischer Mischer (48) vorgesehen ist, mit dem die Ausgangskomponenten beim Durchströmen des Austragsrohrs (42) durch den statischen Mischer (48) durchmischbar sind.

14. Verfahren zum Mischen und Austreiben einer Paste **gekennzeichnet durch** die Schritte:
ein Druckgas wird aus einer Druckgaspatrone (30) durch einen Anschluss (26) eines Hohlzylinders (1) in den Hohlzylinder (1) geleitet, wobei ein Druckbehälter (3, 4) die auf den Wänden des Hohlzylinders (1) lastende Kraft des Druckgases aufnimmt und ein Kolben (8) mit Stößeln (10) im Hohlzylinder (1) durch das Druckgas in Richtung einer Zweikomponentenkartusche (2) vorangetrieben wird;
die vorangetriebenen Stößel (10) des Kolbens (8) treiben Förderkolben (12, 14) in zumindest zwei Innenräume (16, 18) der Zweikomponentenkartusche (2) voran, wobei die Ausgangskomponenten aus den Innenräumen (16, 18) der Zweikomponentenkartusche (2) durch die zumindest zwei Austragsöffnungen (40) der Innenräume (16, 18) ausgetrieben werden,
wobei der Fluss der Ausgangskomponenten durch ein geschlossenes Klappenventil (50) in Fließrichtung hinter den Austragsöffnungen (40) gestoppt wird und durch eine manuellen Bedienung eines Bedienelements (58, 60) ein Joch oder ein Bogen (52) gedreht wird und dabei die mit dem Joch oder dem Bogen (52) verbundene Drehachse (51) des Klappenventils (50) gedreht wird, so dass das Klappenventil (50) geöffnet wird und die Ausgangskomponenten und/oder deren Mischung durch das geöffnete Klappenventil (50) strömen und nach einem Mischen der Ausgangskomponenten die Mischung appliziert wird, wobei durch die manuelle Bedienung des Bedienelements (58, 60) ein Gestänge (54, 56) bewegt wird und durch die Bewegung das Joch oder der Bogen (52) gedreht wird.

15. Verfahren nach Anspruch 14, **dadurch gekennzeichnet, dass**
zur Umsetzung des Verfahrens eine Pasten-Applikationsvorrichtung nach einem der Ansprüche 1 bis 13 verwendet wird.

16. Verfahren nach einem der Ansprüche 14 oder 15, **dadurch gekennzeichnet, dass**
das Klappenventil (50) durch die Einwirkung einer Kraft eines Rückstellelements (62), insbesondere einer elastischen Feder (62), und/oder durch eine manuelle Bedienung des Bedienelements (58, 60) in zur Bedienung zum Öffnen des Klappenventils (50) umgekehrter Richtung geschlossen wird.

## Claims

1. Paste application device for the storage of two starting components, for the mixing of the starting components to form a paste, and for the application of the paste, comprising
a two-component cartridge (2) comprising two internal spaces (16, 18), two feed plungers (12, 14) that can be shifted in the internal spaces (16, 18) and form borders of the internal spaces (16, 18) on a first side of the two-component cartridge (2), and at least two dispensing openings (40) by means of which the internal spaces (16, 18) are open on a second side of the two-component cartridge (2) that is opposite from the first side,
a hollow cylinder (1) made of a plastic material having an open front face and a partially closed front face, in which is situated an axially mobile plunger (8) that has at least two pestles (10) attached to it, whereby the at least two pestles (10) are aligned in the direction of the open front face, whereby the hollow cylinder (1) is arranged such as to touch, by the open front face, axially against the first side of the two-component cartridge (2) and whereby the plunger (8) closes off in gas-tight manner against the internal walls of the hollow cylinder (1), whereby the two-component cartridge (2) and the hollow cylinder (1) are arranged in a pressure vessel (3, 4),
whereby the hollow cylinder (1) comprises, on the partially closed front face, a connector (26) for a compressed gas cartridge (30) that extends through an opening in the pressure vessel (3, 4),
whereby a flap valve (50) that can be rotated manually is arranged downstream of the dispensing openings (40) or a dispensing tube (42) can be attached to a flap valve (50) that can be rotated manually, whereby the volume flow of the starting components through the dispensing openings (40) can be regulated with the arranged flat valve (50),
whereby the flap valve (50) can be operated by means of a rod assembly (54, 56) of the paste application device, whereby the rod assembly (54, 56) is supported such as to be mobile in a main direction with respect to the hollow cylinder (1) and is supported in at least one direction that is perpendicular to the main direction with a tolerance of at least 0.3 mm.

2. Paste application device according to claim 1, **characterised in that**
the rod assembly (54, 56) has a two-part structure, whereby the two parts of the rod assembly (54, 56) are or can be connected to each other by means of an attachment means (55), in particular by means of a bolt, slider (55) or a snap-in element, preferably are or can be connected to each other in a form-fitting manner.

3. Paste application device according to claim 2, **characterised in that**
a first part (54) of the rod assembly (54, 56) is connected to the dispensing tube (42) and a second part (56) of the rod assembly (54, 56) is connected to the hollow cylinder (1).

4. Paste application device according to any one of the claims 1, 2 or 3,
**characterised in that**
at least one restoring element (62) acts on the rod assembly (54, 56) and/or the flap valve (50) such that the restoring element (62) rotates the flap valve (50) into the closed position, whereby the at least one restoring element (62) can preferably be tensioned by pulling the trigger (58, 60).

5. Paste application device according to any one of the preceding claims,
**characterised in that**
the paste application device comprises a trigger (58, 60) that can be operated manually, by means of which the rod assembly (54, 56) can be moved in the main direction and the flap valve (50) can be operated by this means.

6. Paste application device according to claim 5, **characterised in that**
the trigger (58, 60) comprises two levers (58, 60) that are situated at a distance from each other and between which at least one finger of a hand can reach such that the trigger (58, 60) can be moved in both direction by the at least one finger of the hand, whereby the first lever (58) is operated by pulling the trigger (58, 60) and thus opens the flap valve (50), and the second lever (60) is operated by a reverse pushing of the trigger (58, 60) and thus closes the flap valve (50).

7. Paste application device according to claim 5 or 6, **characterised in that**
the trigger (58, 60) transitions the flap valve (50) from the closed to the maximally opened state by means of a stroke of maximally 100 mm, preferably by a stroke of between 2 mm and 40 mm, particularly preferably by a stroke of between 5 mm and 15 mm.

8. Paste application device according to claim 6, **characterised in that**
the restoring element (62) is a compression spring (62) that is supported against a part of a housing (34) that is connected to the hollow cylinder (1).

9. Paste application device according to any one of the preceding claims,
**characterised in that**
a yoke or an arch (34) is attached to the rotary axis (51) of the flap valve (50) in a force-locked or form-fitting manner and is connected to the rod assembly (54, 56) by means of an axle.

10. Paste application device according to claim 9, **characterised in that**
the axle, by means of which the yoke or the arch (34) is connected to the rod assembly (54, 56), is arranged parallel with respect to the rotary axis (51) of the flap valve (50), whereby preferably said axle is arranged on the vertex of the arch (34) or yoke.

11. Paste application device according to any one of the preceding claims,
**characterised in that**
the paste application device has a handle (36) arranged on it by means of which the paste application device can be held with one hand, whereby the rod assembly (54, 56) can be operated with the same hand, in particular by means of the trigger (58, 60), and thus the flap valve (50) can be opened and closed with the same hand.

12. Paste application device according to claim 13, **characterised in that**
in the closed state of the flap valve (50), the trigger (58, 60) is situated at a distance from the handle (36) of maximally 30 mm, and **in that** the trigger (58, 60) touches against the handle (36) or is situated at a distance of no more than 10 mm in the maximally opened state of the flap valve (50).

13. Paste application device according to any one of the preceding claims,
**characterised in that**
the flap valve (50) is arranged in a conduit that is formed by a dispensing tube (42), whereby it is preferred to additionally provide a static mixer (48) in the dispensing tube (42), by means of which the starting components can be mixed while flowing through the dispensing tube (42) and through the static mixer (48).

14. Method for mixing and dispensing a paste, **characterised by** the steps of:
guiding a compressed gas from a compressed gas cartridge (30) through a connector (26) of a hollow cylinder (1) into the hollow cylinder (1), whereby a pressure vessel (3, 4) takes up the force of the compressed gas that bears on the walls of the hollow cylinder (1), and the compressed gas propels a plunger (8) with pestles (10) in the hollow cylinder (1) in the direction of a two-component cartridge (2);
the propelled pestles (10) of the plunger (8) drive feed plungers (12, 14) into at least two internal spaces (16, 18) of the two-component cartridge (2), whereby the starting components are expelled from the internal spaces (16, 18) of the two-component cartridge (2) through the at least two dispensing openings (40) of the internal spaces 16, 18);
whereby the flow of the starting components is stopped by a closed flap valve (50) downstream of the dispensing openings (40) and a yoke or an arch (52) is rotated through a manual operation of an operating element (58, 60) and, in the process, the rotary axis (51) of the flap valve (50) connected to the yoke or the arch (52) is being rotated such that the flap valve (50) is being opened and the starting components and/or the mixtures thereof flow through the open flap valve (50), and, after a mixing of the starting components, the mixing is being applied;
whereby the manual operation of the operating element (58, 60) moves a rod assembly (54, 56) and said motion rotates the yoke or the arch (52).

15. Method according to claim 14, **characterised in that** a paste application device according to any one of the claims 1 to 13 is used to implement the method.

16. Method according to any one of the claims 14 or 15, **characterised in that** the flap valve (50) is being closed by the action of a force of a restoring element (62), in particular of an elastic spring (62), and/or through a manual operation of the operating element (58, 60) in the direction that is reverse to the direction for operation for opening the flap valve (50).

## Revendications

1. Dispositif d'application de pâte pour stocker des deux composantes de départ pour le mélange de composantes de départ en une pâte et pour appliquer la pâte, présentant :
une cartouche bi-composantes (2) présentant deux espaces intérieurs (16, 18), deux pistons de transport (12, 14) mobiles dans les espaces intérieurs (16, 18) qui délimitent les espaces intérieurs (16, 18) sur un premier côté de la cartouche bi-composantes (2), et au moins deux ouvertures de distribution (40) à travers lesquelles les espaces intérieurs (16, 18) sont ouverts sur un second côté de la cartouche bi-composantes (2) opposé au premier côté,
un cylindre creux (1) fabriqué dans un plastique avec une face avant ouverte et une face avant fermée par tronçons, dans lequel un piston (8) mobile axialement est présent avec deux poussoirs (10) fixés dessus, dans lequel les au moins deux poussoirs (10) sont alignés en direction de la face avant ouverte, dans lequel le cylindre creux (1) est disposé en reposant avec la face avant ouverte axialement sur le premier côté de la cartouche bi-composantes (2) et dans lequel le piston (8) finit en étanchéité aux gaz avec les parois intérieures du cylindre creux (1), dans lequel la cartouche bi-composantes (2) et le cylindre creux (1) sont disposés dans un récipient sous pression (3, 4),
dans lequel le cylindre creux (1) présente sur la face avant fermée par tronçons un raccord (26) pour une cartouche de gaz pressurisé (30) qui s'étend à travers une ouverture dans le récipient sous pression (3, 4),
dans lequel dans le sens d'écoulement, un clapet (50) pouvant être tourné manuellement est disposé, ou bien un tube de distribution (42) avec un clapet (50) pouvant être tourné manuellement est à fixer, derrière les ouvertures de distribution (40), dans lequel le débit volumique des composantes de départ se régule par le clapet (50) placé, par les ouvertures de distribution (40),
dans lequel le clapet (50) peut être actionné par le biais d'une tringlerie (54, 56) du dispositif d'application de pâte, dans lequel la tringlerie (54, 56) est disposée dans un sens principal en étant mobile par rapport au cylindre creux (1) et la tringlerie est disposée au moins dans un sens vertical au sens principal avec un jeu d'au moins 0,3 mm.

2. Dispositif d'application de pâte selon la revendication 1, **caractérisé en ce que** la tringlerie (54, 56) est constituée de deux parties, dans lequel les deux parties de la tringlerie (54, 56) sont reliées ou peuvent être reliées entre elles, de préférence sont reliées ou peuvent être reliées entre elles par complémentarité de formes, par le biais d'un moyen de fixation (55), en particulier par un boulon, un poussoir (55) ou un élément d'encliquetage.

3. Dispositif d'application de pâte selon la revendication 2, **caractérisé en ce qu'**une première partie (54) de la tringlerie (54, 56) est reliée au tube de distribution (42) et une seconde partie (56) de la tringlerie (54, 56) est reliée au cylindre creux (1).

4. Dispositif d'application de pâte selon l'une des revendications 1, 2 ou 3, **caractérisé en ce qu'**au moins un élément de rappel (62) agit sur la tringlerie (54, 56) et/ou le clapet (50), de sorte que l'élément de rappel (62) tourne le clapet (50) dans la position fermée, dans lequel de préférence, l'au moins un élément de rappel (62) se tend en tirant la détente (58, 60).

5. Dispositif d'application de pâte selon l'une des revendications précédentes,
**caractérisé en ce que**
le dispositif d'application de pâte comprend une détente (58, 60) pouvant être actionnée manuellement avec laquelle la tringlerie (54, 56) est mobile dans le sens principal et le clapet (50) peut ainsi être utilisé.

6. Dispositif d'application de pâte selon la revendication 5, **caractérisé en ce que** la détente (58, 60) présente deux leviers (58, 60) distants l'un de l'autre, entre lesquels au moins un doigt d'une main peut s'engager, de sorte que la détente (58, 60) est mobile dans les deux sens par l'au moins un doigt de la main, dans lequel le premier levier (58) est actionné en tirant la détente (58, 60) et ouvre ainsi le clapet (50) et le second levier (60) est actionné en poussant la détente (58, 60) dans le sens inverse et ferme ainsi le clapet (50).

7. Dispositif d'application de pâte selon la revendication 5 ou 6, **caractérisé en ce que**
la détente (58, 60) fait passer le clapet (50) de l'état fermé à l'état ouvert maximal avec une course maximale de 100 mm, de préférence avec une course entre 2 mm et 40 mm, particulièrement de préférence avec une course entre 5 mm et 15 mm.

8. Dispositif d'application de pâte selon la revendication 6, **caractérisé en ce que** l'élément de rappel (62) est un ressort à pression (62) qui s'appuie sur une partie d'un logement (34) qui est reliée au cylindre creux (1).

9. Dispositif d'application de pâte selon l'une des revendications précédentes, **caractérisé en ce**
**qu'**une branche ou un coude (34) est fixé(e) par adhérence de forces ou complémentarité de formes sur le pivot (51) du clapet (50), lequel ou laquelle est relié(e) à la tringlerie (54, 56) par un axe.

10. Dispositif d'application de pâte selon la revendication 9, **caractérisé en ce que** l'axe avec lequel la branche ou le coude (34) est fixé(e) à la tringlerie (54, 56), est disposé(e) parallèlement au pivot (51) du clapet (50), dans lequel de préférence, cet axe est disposé sur le sommet du coude (34) ou de la branche.

11. Dispositif d'application de pâte selon l'une des revendications précédentes, **caractérisé en ce**
**qu'**une poignée (36) est disposée sur le dispositif d'application de pâte, avec laquelle le dispositif d'application de pâte peut être tenu d'une main, dans lequel la tringlerie (54, 56), en particulier par le biais de la détente (58, 60), peut être utilisée avec la même main et ainsi le clapet (50) s'ouvre et se ferme avec la même main.

12. Dispositif d'application de pâte selon la revendication 13, **caractérisé en ce que**
la détente (58, 60), lorsque le clapet (50) est fermé, est éloignée au maximum de 30 mm de la poignée (36) et la détente (58, 60), lorsque le clapet (50) est ouvert au maximum, repose sur la poignée (36) ou n'est pas distante de plus de 10 mm.

13. Dispositif d'application de pâte selon l'une des revendications précédentes, **caractérisé en ce que**
le clapet (50) est disposé dans une ligne qui est formée par un tube de distribution (42), dans lequel de préférence un mélangeur statique (48) est prévu en plus dans le tube de distribution (42) avec lequel les composantes de départ peuvent être mélangées par le mélangeur statique (48) lorsqu'elles passent à travers le tube de distribution (42).

14. Procédé de mélange et d'expulsion d'une pâte, **caractérisé par** les étapes suivantes :
un gaz pressurisé est dirigé hors d'une cartouche de gaz pressurisé (30) à travers un raccord (26) d'un cylindre creux (1) jusque dans le cylindre creux (1), dans lequel un récipient sous pression (3, 4) absorbe la force du gaz pressurisé pesant sur les parois du cylindre creux (1) et un piston (8) avec des poussoirs (10) dans le cylindre creux (1) est avancé en direction d'une cartouche bi-composantes (2) par le gaz pressurisé ;
les poussoirs (10) avancés du piston (8) font avancer les pistons de transport (12, 14) dans au moins deux espaces intérieurs (16, 18) de la cartouche bi-composantes (2), dans lequel les composantes de départ sont expulsées des espace intérieurs (16, 18) de la cartouche bi-composantes (2) par les au moins deux ouvertures de distribution (40) des espaces intérieurs (16, 18),
dans lequel le flux des composantes de départ est arrêté derrière les ouvertures de distribution (40) dans le sens d'écoulement par un clapet (50) fermé, et par un actionnement manuel d'un élément de commande (58, 60), une branche ou un coude (52) est tourné(e) et en cela, le pivot (51) du clapet (50) relié à la branche ou au coude est tourné, de sorte que le clapet (50) est ouvert et les composantes de départ et/ou leur mélange s'écoulent à travers le clapet (50) ouvert et après un mélange des composantes de départ, le mélange est appliqué, dans lequel une tringlerie (54, 56) est actionnée par l'actionnement manuel de l'élément de commande (58, 60) et la branche ou le coude (52) est tourné(e) par le mouvement.

15. Procédé selon la revendication 14, **caractérisé en ce que** pour mettre en oeuvre le procédé, un dispositif d'application de pâte est employé selon l'une des revendications 1 à 13.

16. Procédé selon la revendication 14 ou 15, **caractérisé en ce que** le clapet (50), par l'effet d'une force d'un élément de rappel (62), en particulier d'un ressort élastique (62), et/ou par un actionnement manuel de l'élément de commande (58, 60), est fermé dans le sens opposé à l'actionnement pour ouvrir le clapet (50).
